(19) Europäisches Patentamt
European Patent Office
Office européen des brevets

(11) **EP 4 219 004 A1**

(12) **EUROPEAN PATENT APPLICATION**
published in accordance with Art. 153(4) EPC

(43) Date of publication:
**02.08.2023 Bulletin 2023/31**

(21) Application number: **21879414.7**

(22) Date of filing: **13.10.2021**

(51) International Patent Classification (IPC):
**B01J 23/888** (2006.01)  **C07C 5/333** (2006.01)
**C07C 15/46** (2006.01)  **C07C 5/32** (2006.01)

(52) Cooperative Patent Classification (CPC):
**B01J 23/888; C07C 5/32; C07C 5/333;
C07C 15/46;** Y02P 20/52

(86) International application number:
**PCT/CN2021/123478**

(87) International publication number:
**WO 2022/078371 (21.04.2022 Gazette 2022/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.10.2020  CN 202011099425**

(71) Applicants:
• **China Petroleum & Chemical Corporation
Beijing 100728 (CN)**
• **Shanghai Research Institute of Petrochemical
Technology, SINOPEC
Shanghai 201208 (CN)**

(72) Inventors:
• **SONG, Lei**
**Shanghai 201208 (CN)**
• **MIAO, Changxi**
**Shanghai 201208 (CN)**
• **ZHU, Min**
**Shanghai 201208 (CN)**
• **WEI, Chunling**
**Shanghai 201208 (CN)**
• **XU, Yongfan**
**Shanghai 201208 (CN)**

(74) Representative: **karo IP
karo IP Patentanwälte
Kahlhöfer Rößler Kreuels PartG mbB
Postfach 32 01 02
40416 Düsseldorf (DE)**

(54) **IRON-POTASSIUM-CERIUM-BASED COMPOSITE OXIDE CATALYST, AND PREPARATION AND APPLICATION THEREOF**

(57) Disclosed are an iron-potassium-cerium-based composite oxide catalyst, its preparation and application thereof, the catalyst comprising, in addition to the metal elements Fe, K and Ce, a metal element M that is at least one selected from the group consisting of Group IIA metal elements, Group VIB metal elements other than Cr and Group IVA metal elements, wherein the catalyst has a total alkali content of 0.32-0.46 mmol/g, and a strong alkali content of 0.061-0.082 mmol/g. When used for dehydrogenation of alkyl aromatics, the catalyst shows high selectivity, high catalytic activity and high stability, provides less by-products, and has the characteristics of low material consumption and low power consumption, even at a low dehydrogenation temperature and an ultralow steam-to-oil ratio.

Fig. 1

**Description**

**Cross Reference to Related Applications**

The present application claims priority of a Chinese patent application No. 202011099425.4, titled "a catalyst for producing styrene from ethylbenzene by dehydrogenation with low toluene by-product, preparation and application of the same, and method for dehydrogenation of ethylbenzene", filed on October 14, 2020, the contents of which are hereby incorporated by reference in their entirety.

**Technical Field**

The present application relates to the technical field of dehydrogenation catalysts, particularly to an iron-potassium-cerium-based composite oxide catalyst, its preparation and application thereof.

**Background Art**

Ethylbenzene catalytic dehydrogenation is a leading technical route for producing styrene inside and outside China, and its production capacity accounts for about 85% of the total production capacity of styrene. The main reaction of ethylbenzene dehydrogenation is $C_6H_5\text{-}C_2H_5 \rightarrow C_6H_5CH=CH_2+H_2+124KJ/mol$, which is a strong endothermic reaction. Toluene and benzene are main byproducts, the byproduct benzene needs to be separated by a rectification unit and then returned to an ethylbenzene unit for recycling, and the toluene with low economic value can only be sold as a byproduct. Therefore, by reducing the production of benzene and toluene, the utilization of raw materials can be improved, and the material consumption of the plant can be reduced.

One of the key points of the ethylbenzene dehydrogenation is the catalyst for producing styrene by ethylbenzene dehydrogenation, which basically comprises a main catalyst, a cocatalyst, a pore-forming agent, a reinforcing agent and the like. The Fe-K-Ce series catalysts developed in early 1980s show a greatly improved activity while maintaining the stability of the catalyst and avoiding the pollution of chromium oxides to the environment, and thus are employed by styrene manufacturers all over the world.

CN104096568A discloses a composite oxide catalyst for producing styrene by ethylbenzene dehydrogenation, comprising oxides of iron, potassium, magnesium, cerium and molybdenum in the following percentage by mass: 1) 68-75% of iron oxide, calculated as $Fe_2O_3$; 2) 8-13% of potassium oxide, calculated as $K_2O$; 3) 0.5-6% of magnesium oxide, calculated as MgO; 4) 8-15% of cerium oxide, calculated as $CeO_2$; 5) 1-6% of molybdenum oxide, calculated as $MoO_3$; and 6) 0.5-10% of a binder; wherein the precursor of the iron oxide comprises anhydrous iron oxide (i.e. iron oxide red) and hydrous iron oxide (i.e. iron oxide yellow), and the mass ratio of the iron oxide red to the iron oxide yellow is: iron oxide red : iron oxide yellow = 4.5-5.5 : 1; the precursor of the potassium oxide is potassium carbonate; the magnesium oxide is commercially available magnesia; the precursor of the cerium oxide is selected from cerium nitrate and nano cerium oxide, and the content of the nano cerium oxide accounts for 10-50% by mass, preferably 25-40% by mass, of the total amount of the cerium oxide; the precursor of molybdenum oxide is ammonium molybdate $(NH_4)_6Mo_7O_{24}\cdot4H_2O$; the binder may be at least one selected from kaolin, diatomite, cement, and the like. However, the catalyst has a low conversion of ethylbenzene, which is below 65%, and the selectivity of styrene is also low.

The amount of the by-product is an important index for evaluating the performance of the catalyst, and under the same other conditions, catalysts with few by-products and good styrene selectivity are preferably used in styrene dehydrogenation processes. The catalysts disclosed in published U.S. patent No. US5190906A and US4804799A also have the problem of low styrene selectivity, and the styrene selectivity of the isothermal bed dehydrogenation reaction is typically lower than 95.0%, the total content of benzene and toluene in the product is higher than 4%, the material consumption is high, and the difficulty of subsequent separation is increased.

Published U.S. patent No. US6177602A discloses a noble metal-containing iron oxide-based catalyst, with which high selectivity can be obtained and by-products of benzene and toluene are less, but the cost of the catalyst is high duce to the use of a noble metal, which causes a difficulty in industrial application.

Industrial plants for producing styrene by ethylbenzene dehydrogenation in the world mostly have a scale of one hundred thousand tons per year or higher, with the maximum scale being eight hundred thousand tons per year, and employ a high reaction temperature, typically 620 °C or higher, and a high steam-to-oil ratio, typically 1.3 (wt) or higher. High byproducts production, material consumption and energy consumption are always a difficult problem which troubles styrene manufacturers. Therefore, the development of a dehydrogenation catalyst with high selectivity and few byproducts to significantly reduce the material consumption and energy consumption is a tough problem to be solved for the production of styrene by ethylbenzene dehydrogenation at present.

## Disclosure of the Invention

An object of the present application is to provide an iron-potassium-cerium-based composite oxide catalyst its preparation and application thereof, which catalyst, when used for dehydrogenation of alkyl aromatics, shows a high selectivity, catalytic activity and stability even at a low reaction temperature (e.g. not higher than 620 °C) and an ultralow steam-to-oil ratio, and has the advantages of less benzene and toluene byproducts, low material consumption and low energy consumption.

To achieve the above object, in one aspect, the present application provides an iron-potassium-cerium-based composite oxide catalyst comprising a metal element M, in addition to the metal elements Fe, K, and Ce, that is at least one selected from the group consisting of Group IIA metal elements, Group VIB metal elements other than Cr, and Group IVA metal elements, wherein the catalyst has a total alkali content of 0.32 to 0.46 mmol/g, and a strong alkali content of 0.061 to 0.082 mmol/g.

In another aspect, there is provided a method for producing the iron-potassium-cerium-based composite oxide catalyst of the present application, comprising mixing an Fe source, a K source, a Ce source, an M source, optionally a source of a Group IVB metal element, optionally a source of a Group VA metal element, and optionally a ferrite, with a pore-forming agent and a solvent, and shaping, optionally drying and/or calcining, to obtain the catalyst.

In still another aspect, there is provided the use of the iron-potassium-cerium-based composite oxide catalyst of the present application in the dehydrogenation of alkyl aromatics.

In yet another aspect, the present application provides a process for the dehydrogenation of alkyl aromatics, comprising the step of contacting an alkyl aromatic hydrocarbon with the iron-potassium-cerium-based composite oxide catalyst of the present application under dehydrogenation conditions.

When used for the dehydrogenation of alkyl aromatics, the catalyst of the present application shows strong catalytic activity, high selectivity and good stability, even at a low reaction temperature and an ultralow steam-to-oil ratio, and has the advantages of low material consumption and low energy consumption.

Other characteristics and advantages of the present application will be described in detail in the detailed description hereinbelow.

## Brief Description of the Drawings

The drawings, forming a part of the present description, are provided to help the understanding of the present application, and should not be considered to be limiting. The present application can be interpreted with reference to the drawings in combination with the detailed description hereinbelow. In the drawings:

Fig. 1 shows the $CO_2$-TPD patterns of the catalyst obtained in Example 1 of the present application before and after reaction.

Fig. 2 shows the $H_2$-TPR patterns of the catalysts obtained in Example 1 of the present application and Comparative Example 9.

Figs. 3A to 3D show the SEM images of the catalysts obtained in Example 1 of the present application and Comparative Example 9 before and after reaction.

## Detailed Description of the Invention

The present application will be further described hereinafter in detail with reference to the drawing and specific embodiments thereof. It should be noted that the specific embodiments of the present application are provided for illustration purpose only, and are not intended to be limiting in any manner.

Any specific numerical value, including the endpoints of a numerical range, described in the context of the present application is not restricted to the exact value thereof, but should be interpreted to further encompass all values close to said exact value, for example all values within ±5% of said exact value. Moreover, regarding any numerical range described herein, arbitrary combinations can be made between the endpoints of the range, between each endpoint and any specific value within the range, or between any two specific values within the range, to provide one or more new numerical range(s), where said new numerical range(s) should also be deemed to have been specifically described in the present application.

Unless otherwise stated, the terms used herein have the same meaning as commonly understood by those skilled in the art; and if the terms are defined herein and their definitions are different from the ordinary understanding in the art, the definition provided herein shall prevail.

In the present application, unless indicated otherwise, the pressures given are gauge pressures.

In the present application, the total alkali content and strong alkali content of the catalyst are measured according to the carbon dioxide-temperature programmed desorption method ($CO_2$-TPD method).

In the context of the present application, in addition to those matters explicitly stated, any matter or matters not mentioned are considered to be the same as those known in the art without any change. Moreover, any of the embodiments described herein can be freely combined with another one or more embodiments described herein, and the technical solutions or ideas thus obtained are considered as part of the original disclosure or original description of the present application, and should not be considered to be a new matter that has not been disclosed or anticipated herein, unless it is clear to the person skilled in the art that such a combination is obviously unreasonable.

All of the patent and non-patent documents cited herein, including but not limited to textbooks and journal articles, are hereby incorporated by reference in their entirety.

As described above, in the first aspect, the present application provides an iron-potassium-cerium-based composite oxide catalyst, comprising, in addition to the metal elements Fe, K and Ce, a metal element M that is at least one selected from the group consisting of Group IIA metal elements, Group VIB metal elements other than Cr, and Group IVA metal elements, wherein the catalyst has a total alkali content of 0.32 to 0.46 mmol/g, and a strong alkali content of 0.061 to 0.082 mmol/g.

In a preferred embodiment, the metal element M is a combination of at least two selected from the group consisting of Group IIA metal elements, Group VIB metal elements other than Cr, and Group IVA metal elements, preferably a combination of at least one Group IIA metal element, at least one Group VIB metal element other than Cr, and at least one Group IVA metal element.

In a preferred embodiment, the Group IIA metal element comprised in the catalyst is not Mg, more preferably Sr.

In a preferred embodiment, the Group VIB metal element comprised in the catalyst is not Cr or Mo, preferably W.

In a preferred embodiment, the Group IVA metal element comprised in the catalyst is selected from Ge, Sn, and Pb, or a combination thereof.

In a preferred embodiment, after 1500 hours of reaction under conditions including a pressure of -45 kPa, a mass space velocity of ethylbenzene of 0.75 $h^{-1}$, a temperature of 600 °C, and a weight ratio of water to ethylbenzene of 0.9, the retention rate of the crushing strength of the catalyst is 80% or higher.

In a preferred embodiment, after 1500 hours of reaction under conditions including a pressure of -45 kPa, a mass space velocity of ethylbenzene of 0.75 $h^{-1}$, a temperature of 600 °C, and a weight ratio of water to ethylbenzene of 0.9, the retention rate of the total alkali content of the catalyst is 82% or higher, and the retention rate of the strong alkali content of the catalyst is 80% or higher.

In a preferred embodiment, the catalyst has a reduction completion temperature of 730 °C or higher according to the $H_2$-TPR test.

In a preferred embodiment, the catalyst comprises 66-80 wt% of $Fe_2O_3$ based on the total amount of the catalyst, for example, the content by weight of $Fe_2O_3$ can be 66 wt%, 67 wt%, 68 wt%, 69 wt%, 70 wt%, 71 wt%, 72 wt%, 73 wt%, 74 wt%, 75 wt%, 76 wt%, 77 wt%, 78 wt%, 80 wt%, or a value between any two of them.

In a preferred embodiment, the catalyst comprises 2.3-6 wt% of $K_2O$, preferably 2.3-5.5 wt% of $K_2O$, based on the total amount of the catalyst, for example, the content of $K_2O$ can be 2.3 wt%, 2.8 wt%, 3.3 wt%, 3.8 wt%, 4.3 wt%, 4.8 wt%, 5.3 wt%, 5.5 wt%, or a value between any two of them.

In a preferred embodiment, the catalyst comprises 6-12 wt% of $CeO_2$, based on the total amount of the catalyst, for example, 6 wt%, 6.5 wt%, 7 wt%, 7.5 wt%, 8 wt%, 8.5 wt%, 9 wt%, 9.5 wt%, 10 wt%, 10.5 wt%, 11 wt%, 11.5 wt%, 12 wt%, or a value between any two of them.

In a further preferred embodiment, the catalyst comprises 66-80 wt% of $Fe_2O_3$, 2.3-6 wt% of $K_2O$, 6-12 wt% of $CeO_2$, and 2-16 wt% of an oxide of the metal element M, calculated as oxide and based on the total amount of the catalyst. According to the present application, in the catalyst, the metal element M is typically present in the form of an oxide at its highest valency. The inventors of the present application have found that in the above-described content range, the catalyst of the present application shows better catalytic activity, higher selectivity and better stability at a low temperature and an ultralow steam-to-oil ratio, and produces less benzene and toluene. Moreover, conventional catalysts have a $K_2O$ content of higher than 10 wt%, which is easy to be lost during the catalytic dehydrogenation process, thereby reducing the activity of the catalyst; while the $K_2O$ content of the catalyst of the present application is 2.3-6 wt%, which is significantly lower than that of conventional catalysts. With such an extremely low $K_2O$ content, the catalyst of the present application still shows better catalytic activity, higher selectivity and better stability, and produces less benzene and toluene by-products, and thus has higher application advantages as compared to conventional catalysts.

In a particularly preferred embodiment, the oxide of the metal element M is at least one selected from $WO_3$, SrO, or an oxide of a Group IVA metal element. With this preferred embodiment, the catalytic activity, selectivity and stability of the catalyst can be further improved, and a lower amount of benzene and toluene by-products can be obtained.

In the present application, it is preferable that the oxide of the metal element M is at least two selected from $WO_3$, SrO, and an oxide of a Group IVA metal element, and where the oxide of the metal element M is at least two selected from $WO_3$, SrO, and an oxide of a Group IVA metal element, there is no particular limitation on the content of each component in the oxide of the metal element M, and the content of each component may be the same or different.

As can be appreciated that, where the oxide of the metal element M is a combination of two oxides selected

from $WO_3$, SrO, and an oxide of a Group IVA metal element, the contents of the two oxides may be the same or different. Where the contents of the two oxides are different, the respective contents of the two oxides are not particularly limited. Further preferably, the oxide of the metal element M is a combination of $WO_3$, SrO, and an oxide of a Group IVA metal element.

In a preferred embodiment, the catalyst comprises 0.5-5 wt% of $WO_3$, based on the total amount of the catalyst, for example, the content of $WO_3$ may be 0.5 wt%, 1 wt%, 1.5 wt%, 2 wt%, 2.5 wt%, 3 wt%, 3.5 wt%, 4 wt%, 4.5 wt%, 5 wt%, or a value between any two of them.

In a preferred embodiment, the catalyst comprises 0.5-5 wt% SrO, based on the total amount of the catalyst, and the SrO content can be 0.5 wt%, 1 wt%, 1.5 wt%, 2 wt%, 2.5 wt%, 3 wt%, 3.5 wt%, 4 wt%, 4.5 wt%, 5 wt%, or a value between any two of them.

In a preferred embodiment, the catalyst comprises 0.5-5 wt% of the oxide of the Group IVA metal element, based on the total amount of the catalyst, for example, 0.5 wt%, 1 wt%, 1.5 wt%, 2 wt%, 2.5 wt%, 3 wt%, 3.5 wt%, 4 wt%, 4.5 wt%, 5 wt%, or a value between any two of them.

In a further preferred embodiment, the catalyst comprises 67.5-79 wt% of $Fe_2O_3$, 2.3-5.5 wt% of $K_2O$, 6-12 wt% of $CeO_2$, 0.5-5 wt% of $WO_3$, 0.5-5 wt% of SrO, and 0.5-5 wt% of an oxide of a Group IVA metal element, calculated as oxide and based on the total amount of the catalyst. In the above-described content range, the catalyst of the present application shows better catalytic activity, higher selectivity and better stability at a low temperature and an ultralow steam-to-oil ratio, and produces less benzene and toluene by-products.

In a preferred embodiment, the oxide of the Group IVA metal element is at least one selected from the group consisting of $GeO_2$, $SnO_2$, and $PbO_2$. With this preferred embodiment, the catalytic activity and selectivity of the catalyst can be further improved, and a lower amount of benzene and toluene by-products can be obtained.

In the present application, where the oxide of the Group IVA metal element is at least two selected from $GeO_2$, $SnO_2$, and $PbO_2$, there is no particular limitation on the content of each component in the oxide of the Group IVA metal element, and the content of each component may be the same or different.

As can be appreciated that where the oxide of the Group IVA metal element is two selected from $GeO_2$, $SnO_2$, and $PbO_2$, the contents of the two oxides may be the same or different. Where the contents of the two oxides are different, the respective contents of the two oxides are not particularly limited, and preferably, the ratio between the contents of the two oxides is 1 : 0.5-1.5, more preferably 1 : 0.8-1.2, calculated as oxide and based on the total amount of the oxide of the Group IVA metal element.

In a further preferred embodiment, the oxide of the Group IVA metal element is a combination of $GeO_2$, $SnO_2$ and $PbO_2$.

As can be appreciated that where the oxide of the Group IVA metal element is a combination of $GeO_2$, $SnO_2$ and $PbO_2$, the contents of the three oxides may be the same or different. Where the contents of the three oxides are different, the content of each of the three oxides is not particularly limited, and preferably, the ratio between the contents of the three oxides $GeO_2$, $SnO_2$, and $PbO_2$ is 1 : 0.5-1.5 : 0.5-1.5, more preferably 1 : 0.8-1.2 : 0.8-1.2, calculated as oxide and based on the total amount of the oxide of the Group IVA metal element.

In a preferred embodiment, the catalyst further comprises a ferrite, such as manganese ferrite, zinc ferrite, copper ferrite, nickel ferrite, and the like, preferably $ZnFe_2O_4$. The inventors of the present application have found through research that, where a certain amount of Fe element is added in the form of a ferrite, the resulting catalyst has higher activity, better selectivity, higher stability and produces less benzene and toluene by-products compared to the case where the Fe element is simply added in the form of an oxide, for example, the addition of $ZnFe_2O_4$ compared to the addition of an equivalent amount of ZnO and iron oxide.

In a preferred embodiment, the catalyst comprises 0.5-8 wt% of ferrite, preferably $ZnFe_2O_4$, based on the total amount of the catalyst, for example, 0.5 wt%, 1.5 wt%, 2.5 wt%, 3.5 wt%, 4.5 wt%, 5.5 wt%, 6.5 wt%, 7.5 wt%, 8 wt%, or a value between any two of them.

In a further preferred embodiment, the content of the ferrite, such as $ZnFe_2O_4$, in the catalyst is 1-7 wt%, and more preferably 2-6 wt%, to further improve the catalytic activity, selectivity and stability of the catalyst and further reduce the production of benzene and toluene by-products.

In a particularly preferred embodiment, the catalyst comprises 2-6 wt% of $ZnFe_2O_4$, based on the total amount of the catalyst, and the content of $ZnFe_2O_4$ can be, for example, 2 wt%, 2.5 wt%, 3 wt%, 3.5 wt%, 4 wt%, 4.5 wt%, 5 wt%, 5.5 wt%, 6 wt%, or a value between any two of them.

In a preferred embodiment, the catalyst may further comprise other metal promoters, for example, Group IVB metal oxides, such as $HfO_2$, and/or Group VA metal oxides, such as $Sb_2O_5$. Preferably, the catalyst comprises 0.05-0.5 wt% of a Group IVB metal oxide, preferably $HfO_2$, and/or 0.5-1.5 wt% of a Group VA metal oxide, preferably $Sb_2O_5$, based on the total amount of the catalyst.

According to the present application, to further improve the catalytic activity, selectivity and stability of the catalyst at a low temperature and further reduce the production of benzene and toluene by-products, it is preferable that the catalyst does not comprise molybdenum.

According to the present application, to further improve the activity of the catalyst at a low steam-to-oil ratio, and reduce the dehydrogenation temperature, it is preferable that the catalyst does not comprise magnesium.

In a preferred embodiment, the catalyst does not comprise a binder, such as montmorillonite, diatomite, cement, metahalloysite, saponite, kaolin, halloysite, hydrotalcite, sepiolite, rectorite, attapulgite, bentonite, or any combination thereof. The inventors of the present application have found through research that the addition of a binder in the catalyst of the present application is not beneficial to improving the catalytic activity and stability of the catalyst, and this preferred embodiment is more beneficial to improving the catalytic activity and stability of the catalyst.

In a particularly preferred embodiment, the catalyst of the present application comprises Fe element, K element, Ce element, and metal element M, and at least one of $ZnFe_2O_4$, $HfO_2$, and $Sb_2O_5$, the metal element M being at least one selected from W element, Sr element, and Group IVA metal element. Further preferably, the catalyst comprises $ZnFe_2O_4$ and at least one of $HfO_2$ and $Sb_2O_5$. The content of each element is as described above, of which a detailed description is omitted herein for brevity.

The inventors of the present application have found through research that where the iron-potassium-cerium-based composite oxide catalyst comprises the above-defined metal components, and has a total alkali content of 0.32 to 0.46 mmol/g, along with a strong alkali content of 0.061 to 0.082 mmol/g, the catalyst shows strong catalytic activity, high selectivity and good stability even at a low reaction temperature (e.g., not higher than 620 °C) and an ultralow steam-to-oil ratio when used for the dehydrogenation of alkyl aromatics. The above superiority of controlling the total alkali content and the strong alkali content of catalysts for dehydrogenation of alkyl aromatics within the above-described specific ranges is not known in the art before, and therefore there is no particular requirement on the total alkali content and strong alkali content of the catalyst and the stability of the alkali content during the reaction process in prior arts, and the total alkali content of existing catalysts is typically below 0.32 mmol/g.

The catalyst of the present application is provided with a total alkali content and a strong alkali content within the specific range by means of a coordination of specific elements, which allows a reduction of the introduction of low-efficiency substances, so that, when the catalyst is used for dehydrogenation of ethylbenzene, the generation of benzene and toluene can be reduced, the selectivity of styrene and the utilization of raw materials can be improved, and a small alkali content fluctuation before and after reaction can be achieved. For example, the crushing strength of the catalyst obtained in the present application can reach 3.88 Kg/mm, and in an activity evaluation carried out on an isothermal fixed bed under conditions including a pressure of -45 kPa, a liquid mass space velocity of ethylbenzene of 0.75 h$^{-1}$, a reaction temperature of 600 °C and a steam-to-oil ratio (namely the weight ratio of water to ethylbenzene) of 0.9, the total content of benzene and toluene in the product is as low as 2.26%, the selectivity of styrene can reach 97.17%, and the conversion of ethylbenzene can reach 77.6%; after 1500 hours of reaction, the total content of benzene and toluene in the product is as low as 1.93%, the selectivity of styrene can reach 97.99%, and the conversion of ethylbenzene can reach 76.7%; and after 1500 hours of reaction, the retention rate of the total alkali content of the catalyst can reach 94.96%, the retention rate of the strong alkali content of the catalyst can reach 92.41%, and the retention rate of the crushing strength of the catalyst can reach 94.85%, which indicates a good stability.

In a preferred embodiment, the total alkali content of the catalyst is 0.32 to 0.42 mmol/g, preferably 0.324 to 0.397 mmol/g, particularly preferably 0.384 to 0.397 mmol/g, and may be, for example, 0.384 mmol/g, 0.386 mmol/g, 0.388 mmol/g, 0.39 mmol/g, 0.392 mmol/g, 0.394 mmol/g, 0.396 mmol/g, 0.397 mmol/g, or a value between any two of them.

In a preferred embodiment, the strong alkali content the catalyst is 0.061 to 0.080 mmol/g, preferably 0.061 to 0.079 mmol/g, particularly preferably 0.072 to 0.079 mmol/g, and may be, for example, 0.072 mmol/g, 0.074 mmol/g, 0.076 mmol/g, 0.078 mmol/g, 0.079 mmol/g, or a value between any two of them.

In a particularly preferred embodiment, the catalyst of the present application has a total alkali content of 0.384-0.397 mmol/g and a strong alkali content of 0.072-0.079 mmol/g, in which case the catalyst of the present application exhibits better catalytic activity, higher selectivity and better stability, and provides a lower benzene and toluene content in the product, under poor catalytic dehydrogenation conditions.

In a second aspect, there is provided a method for producing the iron-potassium-cerium-based composite oxide catalyst of the present application, comprising mixing an Fe source, a K source, a Ce source, an M source, optionally a source of a Group IVB metal element, optionally a source of a Group VA metal element, and optionally a ferrite, with a pore-forming agent and a solvent, and shaping, optionally drying and/or calcining, to obtain the catalyst; wherein the M source is at least one selected from the group consisting of sources of Group IIA metal elements, sources of Group VIB metal elements other than Cr, and sources of Group IVA metal elements, preferably at least one of a W source, an Sr source, and a Group IVA metal element source, more preferably a combination of at least two of a W source, an Sr source, and a Group IVA metal element source, and particularly preferably a combination of a W source, an Sr source, and at least one Group IVA metal element source.

In the second aspect of the present application, the Group IVB metal element, the Group VA metal element, the ferrite, the Group IIA metal element, the Group VIB metal element other than Cr, the Group IVA metal element, and the like are as defined above, of which a detailed description is omitted herein for brevity.

There is no particular limitation to the mode of mixing in the method of the present application, as long as the Fe source, the K source, the Ce source, the M source, the pore-forming agent, the solvent, the optional Group IVB metal element source, the optional Group VA metal element source, and the optional ferrite can be uniformly mixed.

To further increase the catalytic activity and selectivity of the resulting catalyst and reduce the toluene content of the product at a low temperature, in a preferred embodiment, the method of the present application comprises the steps of:

> 1) mixing an Fe source, a K source, a Ce source, an M source, optionally a source of a Group IVB metal element, and optionally a source of a Group VA metal element with a pore-forming agent;
> 2) mixing the mixture obtained in step 1) with a ferrite, wherein the ferrite is preferably $ZnFe_2O_4$; and
> 3) mixing the mixture obtained in step 2) with a solvent, shaping, and optionally drying and/or calcining to obtain the catalyst.

In such a preferred embodiment, uniform mixing can be facilitated, the catalytic activity, selectivity and stability of the catalyst can be further improved, and the production of benzene and toluene byproducts can be further reduced.

In the preferred embodiment described above, there is no particular requirement on the mixing time of step 1), step 2) and step 3), which can be adjusted within a wide range as long as it enables the materials to be mixed uniformly in the above-described order of addition. Preferably, to save energy consumption while ensuring a uniform mixing of the materials, the mixing time of step 1), step 2) and step 3) is independently 0.1-2h; further preferably, the mixing time of step 1) is 0.1-0.6 h, the mixing time of step 2) is 1-2h, and the mixing time of step 3) is 0.2-1 h.

In the method of the present application, the mode of mixing can be selected within a wide range, and the mixing can be carried out, for example, under stirring conditions. The mixing may also be carried out in a kneader.

In the present application, there is no particular limitation to the selection of the Fe source, which may be any iron-containing compound that can be converted to $Fe_2O_3$ in a subsequent calcining process. Preferably, the Fe source is iron oxide red and/or iron oxide yellow, more preferably a combination of iron oxide red and iron oxide yellow. In such a preferred embodiment, the catalytic activity and selectivity of the catalyst can be further improved, and the production of benzene and toluene byproducts can be further reduced.

In the present application, the ratio of the iron oxide red to the iron oxide yellow can be selected within a wide range, and preferably, the weight ratio, calculated as oxide, of the iron oxide red to the iron oxide yellow is 2-4: 1; for example, it may be 2 : 1, 2.2 : 1, 2.4 : 1, 2.6 : 1, 2.8 : 1, 3 : 1, 3.2 : 1, 3.4 : 1, 3.6 : 1, 3.8 : 1, 4 : 1, or a value between any two of them.

In the present application, there is no particular limitation to the selection of the Ce source, which can be any cerium-containing compound that can be converted to $CeO_2$ in a subsequent calcining process. Preferably, the Ce source is cerium hydroxide and/or cerium acetate. In such a preferred embodiment, the environmental protection requirement (as nitrogen-containing gas will be released during the calcining of cerium nitrate) can be met, and the strength of the resulting catalyst can be further improved.

In the present application, there is no particular limitation to the selection of the K source, which may be any potassium-containing compound that can be converted to $K_2O$ in a subsequent calcining process. Preferably, the K source is potassium carbonate and/or potassium bicarbonate; more preferably potassium carbonate.

In the present application, there is no particular limitation to the selection of the source of the Group VIB metal element, which may be any compound containing a Group VIB metal element that can be converted into a Group VIB metal oxide in a subsequent calcining process, and may be a salt containing a Group VIB metal element and/or an oxide of a Group VIB metal element.

In the present application, there is no particular limitation to the selection of the W source, which may be any tungsten-containing compound that can be converted to $WO_3$ in a subsequent calcining process, and preferably, the W source is at least one selected from the group consisting of ammonium tungstate, ammonium metatungstate, and tungsten trioxide; more preferably ammonium tungstate.

In the present application, there is no particular limitation to the selection of the source of the Group IIA metal element, which may be any compound containing a Group IIA metal element that can be converted into a Group IIA metal oxide in a subsequent calcining process, and may be a salt containing a Group IIA metal element and/or an oxide of a Group IIA metal element.

In the present application, there is no particular limitation to the selection of the Sr source, which may be any strontium-containing compound that can be converted into SrO in a subsequent calcining process, and preferably, the Sr source is strontium carbonate and/or strontium hydroxide.

In the present application, there is no particular limitation to the selection of the source of the Group IVA metal element, which may be any compound containing a Group IVA metal element that can be converted into a Group IVA metal oxide in a subsequent calcining process, and may be a salt containing a Group IVA metal element and/or an oxide of a Group IVA metal element. Preferably, the source of the Group IVA metal element is selected from oxides containing a Group IVA metal element.

In the present application, there is no particular limitation to the selection of the source of the Group IVB metal element, which may be any compound containing a Group IVB metal element that can be converted to a Group IVB metal oxide in a subsequent calcining process, and may be a salt containing a Group IVB metal element and/or an oxide containing a Group IVB metal element.

In the present application, there is no particular limitation to the selection of the Hf source, which may be any salt and/or oxide containing Hf element that can be converted to hafnium oxide in a subsequent calcining process, preferably $HfO_2$.

In the present application, there is no particular limitation to the selection of the source of the Group VA metal element, which may be any compound containing a Group VA metal element that can be converted into a Group VA metal oxide in a subsequent calcining process, and may be a salt containing a Group VA metal element and/or an oxide containing a Group VA metal element.

In the present application, there is no particular limitation to the selection of the Sb source, which may be any salt and/or oxide containing Sb element that can be converted to antimony oxide in a subsequent calcining process, preferably $Sb_2O_5$.

In the present application, there is no particular limitation to the amount of the pore-forming agent added, and to provide a large specific surface area while ensuring a desired strength of the catalyst, it is preferable that the pore-forming agent is added in an amount of 2.2 to 6.3 wt%, preferably 3.8 to 5.6 wt%, relative to the total amount of the Fe source, the K source, the Ce source, the M source, the optional ferrite, the optional Group IVB metal element source, and the optional Group VA metal element source.

In the present application, there is no particular limitation to the type of the pore-forming agent, which may be any of those conventionally used in the art. Preferably, the pore-forming agent is at least one selected from the group consisting of polystyrene, graphite, and cellulose and derivatives thereof. In the present application, the graphite may be selected within a wide range, and it may be natural graphite or artificial graphite with no particular limitation in the present application.

The cellulose and derivatives thereof are preferably at least one of methylcellulose, hydroxymethyl cellulose, ethylcellulose and sodium hydroxymethyl cellulose.

In a preferred embodiment of the present application, the pore-forming agent is at least one selected from the group consisting of graphite, polystyrene (which may be in the form of microspheres), and sodium carboxymethyl cellulose.

In the present application, there is no particular limitation to the amount of the solvent added, as long as the requirements on the mixing of the materials can be satisfied. Preferably, the solvent is added in an amount of 15 to 35 wt%, preferably 22 to 32 wt%, relative to the total weight of the catalyst raw materials.

According to the present application, the solvent may be selected within a wide range, as long as it can provide a desired environment for mixing, and preferably, the solvent is water.

In the present application, there is no particular limitation to the shape of the catalyst, which may be, for example, a pellet, a bar, or the like. The person skilled in the art can form the mixed material obtained in step 3) into various usable sizes according to the requirements in production, for example, the material may be extruded into granules having a diameter of 3 mm and a length of 6 mm, but the present application is not limited thereto.

In a preferred embodiment, the drying conditions of step 3) include: a temperature of 30-145 °C, and a drying time of 1-8 h; more preferably, the drying comprises: drying at 35-95 °C for 0.5-4 hr, and then heating to 95-145 °C and further drying for 0.5-4 hr.

In a preferred embodiment, the calcining conditions of step 3) include: a temperature of 320-960 °C, and a calcining time of 2-8 h. More preferably, the calcining comprises: calcining at 320-750 °C for 1-4 h, and then heating to 750-960 °C and further calcining for 1-4 h.

In a third aspect, there is provided an iron-potassium-cerium-based composite oxide catalyst obtained by the method of the present application.

In a fourth aspect, there is provided the use of the iron-potassium-cerium-based composite oxide catalyst of the present application in the dehydrogenation of alkyl aromatics.

In a fifth aspect, the present application provides a process for the dehydrogenation of alkyl aromatics, comprising the step of contacting an alkyl aromatic hydrocarbon with the iron-potassium-cerium-based composite oxide catalyst of the present application under dehydrogenation conditions.

In a preferred embodiment, the alkyl aromatic hydrocarbon is one or more of $C_8$-$C_{10}$ alkyl benzenes, more preferably ethylbenzene.

When used for the dehydrogenation of alkyl aromatics, the catalyst of the present application shows higher catalytic activity, selectivity and stability, and provides a low content of benzene and toluene byproducts in the product, even at a low temperature and an ultralow steam-to-oil ratio.

In a preferred embodiment, the temperature for the dehydrogenation of the alkyl aromatic hydrocarbon may be 580-620 °C, more preferably 590-610 °C, for example, 590 °C, 595 °C, 600 °C, 605 °C, 610 °C or a value between any two of them.

According to the present application, to further reduce the material consumption, the mass space velocity of the alkyl aromatic hydrocarbon is preferably 0.5-1 $h^{-1}$, more preferably 0.6-0.8 $h^{-1}$, and may be, for example, 0.6 $h^{-1}$, 0.65 $h^{-1}$, 0.7 $h^{-1}$, 0.75 $h^{-1}$, 0.8 $h^{-1}$, or any value between any two values.

According to the present application, to further reduce the energy consumption, preferably, the weight ratio of water to the alkyl aromatic hydrocarbon is 0.7 to 1, more preferably 0.8 to 1, and may be, for example, 0.8, 0.82, 0.84, 0.86, 0.88, 0.9, 0.92, 0.94, 0.96, 0.98, 1, or a value between any two of them.

According to the present application, the pressure for the dehydrogenation of the alkyl aromatic hydrocarbon may be selected within a wide range, preferably the pressure may be from -60 kPa to atmospheric pressure , more preferably from -50 kPa to atmospheric pressure , and even more preferably from -50 kPa to -20 kPa, for example, -50 kPa, -48 kPa, -46 kPa, -44 kPa, -42 kPa, -40 kPa, -38 kPa, -36 kPa, -34 kPa, -32 kPa, -30 kPa, -28 kPa, -26 kPa, -24 kPa, -22 kPa, -20 kPa, or a value between any two of them.

In particularly preferred embodiments, the dehydrogenation conditions may include: a temperature of 580-620 °C, a mass space velocity of the alkyl aromatic hydrocarbon of 0.5-1 $h^{-1}$, a weight ratio of water to the alkyl aromatic hydrocarbon of 0.7-1, and a pressure of -60 kPa to normal pressure; further preferably, the conditions for the dehydrogenation of the alkyl aromatic hydrocarbon include: a temperature of 590-610 °C, a mass space velocity of 0.6-0.8 $h^{-1}$, a weight ratio of water to the alkyl aromatic hydrocarbon of 0.8-1, and a pressure of -50 kPa to atmospheric pressure.

**Examples**

The present application will be further illustrated with reference to the following examples, but the present application is not limited thereto.

In the following examples and comparative examples, the performance of the catalyst was characterized by ethylbenzene conversion, styrene selectivity, and the contents of benzene and toluene in the product, respectively. Specifically, the performance of the catalyst was evaluated in an isothermal fixed bed, of which the process was briefly as follows: deionized water and ethylbenzene were respectively introduced into a preheating mixer through a metering pump, mixed and preheated into a gas state and then passed to a reactor, the reactor was heated using electric heating wires to a preset temperature, the reactor was a stainless steel tube with an inner diameter of 1 inch and was filled with 100 milliliters of a catalyst, the reaction product exiting the reactor was condensed with water, and then the ethylbenzene concentration (by wt%), the styrene concentration (by wt%) and the benzene and toluene concentration (by wt%) in the reaction product were analyzed using a gas chromatograph;

$$\text{Conversion of Ethylbenzene (\%)} = \text{(initial ethylbenzene concentration in the reaction stream (wt\%) - ethylbenzene concentration in the reaction product (wt\%)) / initial ethylbenzene concentration in the reaction stream (wt\%);}$$

and

$$\text{Selectivity of styrene (\%)} = \text{styrene concentration in the reaction product (wt\%) / (initial ethylbenzene concentration in the reaction stream (wt\%) - ethylbenzene concentration in the reaction product (wt\%)).}$$

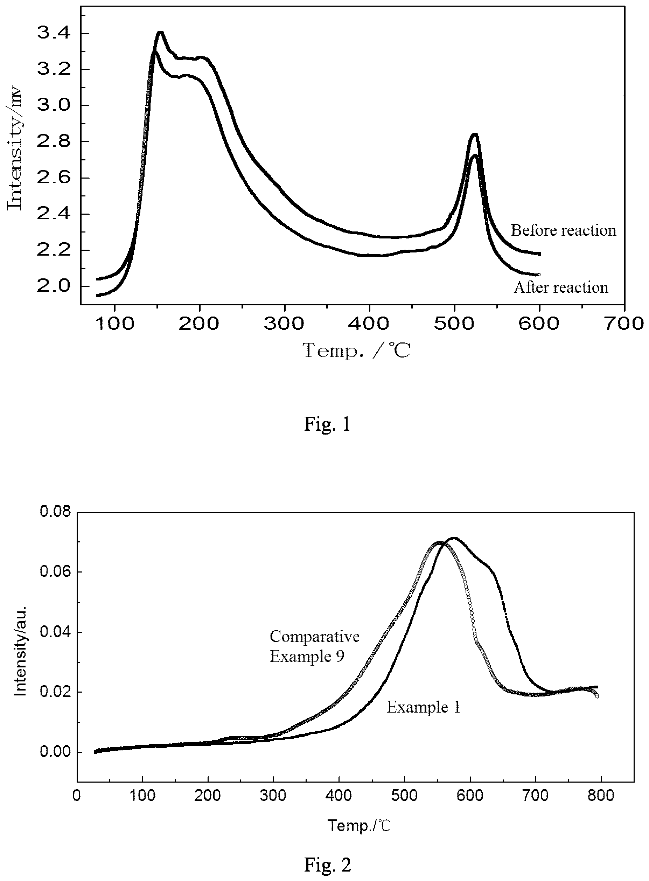

In the following examples and comparative examples, the total alkali content and strong alkali content of the catalyst were analyzed by the carbon dioxide-temperature programmed desorption method, in which 0.1g of the catalyst was activated at 600 °C for 2 hours in a flow of helium gas, then cooled to 80 °C, and subjected to $CO_2$ adsorption until an equilibrium was arrived, purged with helium gas to remove $CO_2$ physically adsorbed, then heated from 80 °C to 600 °C at 10 °C/min by temperature programming method, the $CO_2$-TPD pattern was recorded, and $CO_2$ desorbed was collected using liquid nitrogen at the same time, and the $CO_2$ collected was quantitatively analyzed by gas chromatography. A typical $CO_2$-TPD pattern is shown in Fig. 1, in which the peak present in the low temperature region (80-400 °C) is the desorption peak corresponding to the weak alkali content, the peak in the high temperature region (400-600 °C) is the desorption peak corresponding to the strong alkali content. $CO_2$ desorbed was collected using liquid nitrogen

while recording the $CO_2$-TPD pattern, the amount of the $CO_2$ collected was quantitatively analyzed by gas chromatography, and corresponding weak alkali content and strong alkali content were calculated. The total alkali content is the sum of the weak alkali content and the strong alkali content. The retention rate of the alkali content is the ratio of the alkali content after reaction to the alkali content before reaction.

In the following examples and comparative examples, the electron microscope image of the catalyst was taken on a JSM-35 Scanning Electron Microscope manufactured by Nippon electronics Co., Ltd., at operating acceleration voltages of 15, 25 kV, respectively, a working distance of 20 mm, a magnification of 20,000, and a resolution of 3.5nm.

In the following examples and comparative examples, the measurement of the crushing strength of the catalyst was performed according to HG/T2782-201 1, using a QCY-602 particle strength meter. 40 granular test samples were taken by quartering, and the crushing strength of the sample was calculated as the arithmetic mean of the measured results. The retention rate of the crushing strength is the ratio of the crushing strength after reaction to the crushing strength before reaction.

In the following examples and comparative examples, the change in the reduction temperature of the catalyst was observed using a Temperature Programmed Reduction (TPR) method, in which 50 mg sample of the catalyst was placed in a U-tube quartz reactor, heated to 400 °C under an atmosphere of helium, and then cooled to room temperature, the atmosphere was switched to $H_2/N_2$ reducing gas (with an $H_2$ concentration of 10% by volume) to carry out temperature programmed reduction, and the temperature was raised to 850 °C at a rate of 10 °C/min.

Example 1

55.2 parts by weight of iron oxide red, calculated as $Fe_2O_3$, 20.1 parts by weight of iron oxide yellow, calculated as $Fe_2O_3$, 4.66 parts by weight of potassium carbonate, calculated as $K_2O$, 9.89 parts by weight of cerium acetate, calculated as $CeO_2$, 2.61 parts by weight of ammonium tungstate, calculated as $WO_3$, 2.38 parts by weight of strontium carbonate, calculated as SrO, 1.68 parts by weight of $GeO_2$, 0.32 parts by weight of $HfO_2$, and 5.69 parts by weight of sodium carboxymethyl cellulose (commercially available from Shanghai Changguang Enterprise Development Co. Ltd., premium food products, the same applies below) were stirred in a kneader for 0.5 hours, 3.16 parts by weight of $ZnFe_2O_4$ was added thereto and further stirred for 1.5 hours, then deionized water was added thereto in an amount of 26.3 wt% relative to the total weight of the catalyst raw materials, stirred and mixed for 0.5 hours, the resultant was taken out and extruded into granules having a diameter of 3 mm and a length of 6 mm, the granules were placed in an oven, dried at 55 °C for 2 hours, heated to 135 °C and dried for 3 hours, then placed in a muffle furnace, calcined at 455 °C for 3 hours, then heated to 930 °C and calcined for 3 hours to obtain a catalyst product, of which the composition is shown in Table 1.

The change in the reduction temperature of the catalyst obtained in Example 1 was observed by a Temperature Programmed Reduction (TPR) method, and the $H_2$-TPR pattern obtained is shown in Fig. 2, and it can be seen from Fig. 2 that the reduction completion temperature of the catalyst (i.e., the temperature corresponding to the peak of the curve in the $H_2$-TPR pattern) was 746 °C.

100 ml of the catalyst product obtained in this example was charged into a reactor, and a performance evaluation was performed under conditions of -45 kPa, a mass space velocity of 0.75 h$^{-1}$, 600 °C and a weight ratio of steam to ethylbenzene of 0.9, and the test results obtained after 100 hours and 1500 hours of reaction were shown in Table 5.

The total alkali content and strong alkali content of the catalyst obtained in Example 1 before reaction and after 1500 hours of reaction were analyzed by the carbon dioxide-temperature programmed desorption method, and the $CO_2$-TPD pattern obtained is shown in Fig. 1. In Fig. 1, the peak in the low temperature region is the desorption peak corresponding to the weak alkali content, and the peak in the high temperature region is the desorption peak corresponding to the strong alkali content. $CO_2$ desorbed was collected using liquid nitrogen while recording the $CO_2$-TPD pattern, the amount of the $CO_2$ collected was quantitatively analyzed by gas chromatography, and corresponding weak alkali content and strong alkali content were calculated. The total alkali content is the sum of the weak alkali content and the strong alkali content. The strong alkali content and the total alkali content before and after reaction of the catalyst obtained in Example 1 and the retention rate of the alkali content are shown in Table 4.

The catalyst obtained in Example 1 has a crushing strength of 3.35Kg/mm before reaction, a strength of 2.88Kg/mm after reaction and a retention rate of the strength of 85.97%, as measured by the method of HG/T2782-2011, and the results are shown in Table 4.

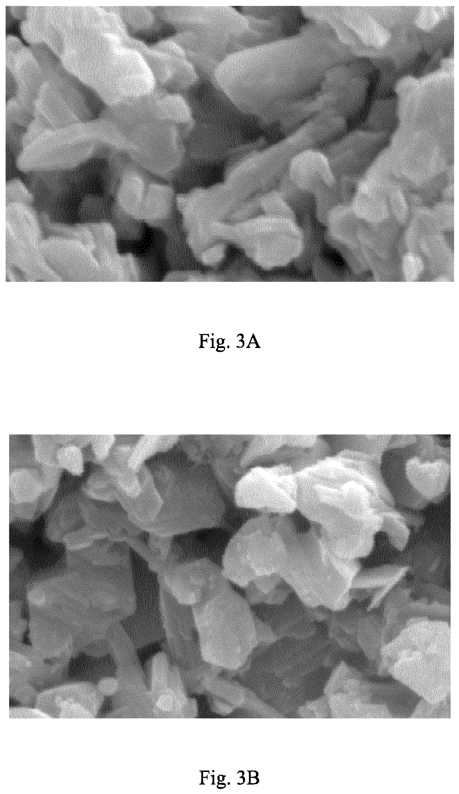

An electron microscope image of the catalyst was taken on a scanning electron microscope, and the scanning electron microscope (SEM) images of the catalyst obtained in Example 1 before and after reaction are shown in Figs. 3A and 3B, respectively.

Comparative Example 1

A catalyst was prepared as described in Example 1, except that $ZnFe_2O_4$ and $GeO_2$ were not added, specifically: 58.01 parts by weight of iron oxide red, calculated as $Fe_2O_3$, 21.12 parts by weight of iron oxide yellow, calculated as

$Fe_2O_3$, 4.9 parts by weight of potassium carbonate, calculated as $K_2O$, 10.39 parts by weight of cerium acetate, calculated as $CeO_2$, 2.74 parts by weight of ammonium tungstate, calculated as $WO_3$, 2.5 parts by weight of strontium carbonate, calculated as SrO, 0.34 parts by weight of $HfO_2$ and 5.69 parts by weight of sodium carboxymethyl cellulose were stirred in a kneader for 2.0 hours, deionized water was added thereto in an amount of 26.3 wt% relative to the total weight of the catalyst raw materials and mixed for 0.5 hours, the resultant was taken out and extruded into granules having a diameter of 3 mm and a length of 6 mm, the granules were placed in an oven, dried at 55 °C for 2 hours, heated to 135 °C and dried for 3 hours, then placed in a muffle furnace, calcined at 455 °C for 3 hours, and then calcined at 930 °C for 3 hours to obtain a catalyst product, of which the composition is shown in Table 1.

The catalyst was evaluated and analyzed as described in Example 1, the results of the alkali content analysis and the strength measurement are shown in Table 4, and the evaluation results are shown in Table 5.

Comparative Example 2

A catalyst was prepared, evaluated and analyzed as described in Example 1, except that $ZnFe_2O_4$ was not added but replaced with iron oxide red, iron oxide yellow and ZnO at an equivalent elemental oxide amount, the relative proportions of the remaining components, the catalyst preparation method and the catalyst evaluation conditions and analysis method were the same as in Example 1. The composition of the catalyst is shown in Table 1, the results of the alkali content analysis and the strength measurement are shown in Table 4, and the evaluation results are shown in Table 5.

Example 2

A catalyst was prepared, evaluated and analyzed as described in Example 1, except that $SnO_2$ was used instead of $GeO_2$. The composition of the catalyst is shown in Table 1, the results of the alkali content analysis and the strength measurement are shown in Table 4, and the evaluation results are shown in Table 5.

Comparative Example 3

A catalyst was prepared, evaluated and analyzed as described in Example 1, except that $SnO_2$ was used instead of $GeO_2$, $ZnFe_2O_4$ was not added but replaced with iron oxide red, iron oxide yellow and ZnO at an equivalent elemental oxide amount, calculated as oxide. The composition of the catalyst is shown in Table 1, the results of the alkali content analysis and the strength measurement are shown in Table 4, and the evaluation results are shown in Table 5.

Example 3

A catalyst was prepared, evaluated and analyzed as described in Example 1, except that $PbO_2$ was used instead of $GeO_2$. The composition of the catalyst is shown in Table 1, the results of the alkali content analysis and the strength measurement are shown in Table 4, and the evaluation results are shown in Table 5.

Comparative Example 4

A catalyst was prepared, evaluated and analyzed as described in Example 1, except that $PbO_2$ was used instead of $GeO_2$, $ZnFe_2O_4$ was not added but replaced with iron oxide red, iron oxide yellow and ZnO at an equivalent elemental oxide amount, calculated as oxide. The composition of the catalyst is shown in Table 1, the results of the alkali content analysis and the strength measurement are shown in Table 4, and the evaluation results are shown in Table 5.

Example 4

A catalyst was prepared, evaluated and analyzed as described in Example 1, except that 0.84 parts by weight of $GeO_2$ and 0.84 parts by weight of $SnO_2$ were used instead of 1.68 parts by weight of $GeO_2$. The composition of the catalyst is shown in Table 1, the results of the alkali content analysis and the strength measurement are shown in Table 4, and the evaluation results are shown in Table 5.

Example 5

A catalyst was prepared, evaluated and analyzed as described in Example 1, except that 0.84 parts by weight

of $GeO_2$ and 0.84 parts by weight of $PbO_2$ were used instead of 1.68 parts by weight of $GeO_2$. The composition of the catalyst is shown in Table 1, the results of the alkali content analysis and the strength measurement are shown in Table 4, and the evaluation results are shown in Table 5.

Example 6

A catalyst was prepared, evaluated and analyzed as described in Example 1, except that 0.84 parts by weight of $SnO_2$ was used and 0.84 parts by weight of $PbO_2$ were used instead of 1.68 parts by weight of $GeO_2$. The composition of the catalyst is shown in Table 1, the results of the alkali content analysis and the strength measurement are shown in Table 4, and the evaluation results are shown in Table 5.

Example 7

A catalyst was prepared, evaluated and analyzed as described in Example 1, except that 0.56 parts by weight of $GeO_2$, 0.56 parts by weight of $SnO_2$, and 0.56 parts by weight of $PbO_2$ were used instead of 1.68 parts by weight of $GeO_2$. The composition of the catalyst is shown in Table 1, the results of the alkali content analysis and the strength measurement are shown in Table 4, and the evaluation results are shown in Table 5.

Example 8

54.01 parts by weight of iron oxide red, calculated as $Fe_2O_3$, 16.9 parts by weight of iron oxide yellow, calculated as $Fe_2O_3$, 2.37 parts by weight of potassium carbonate, calculated as $K_2O$, 11.84 parts by weight of cerium acetate, calculated as $CeO_2$, 4.16 parts by weight of ammonium tungstate, calculated as $WO_3$, 3.35 parts by weight of strontium carbonate, calculated as $SrO$, 0.85 parts by weight of $GeO_2$, 0.06 parts by weight of $HfO_2$, 0.96 parts by weight of $Sb_2O_5$ and 5.69 parts by weight of sodium carboxymethyl cellulose were stirred in a kneader for 1 hour, 5.5 parts by weight of $ZnFe_2O_4$ were added thereto and further stirred for 2 hours, deionized water was added thereto in an amount of 15 wt% relative to the total weight of the catalyst raw materials, stirred and mixed for 1 hour, the resultant was taken out and extruded into granules having a diameter of 3 mm and a length of 6 mm, the granules were placed in an oven, dried at 30 °C for 4 hours, then dried at 95 °C for 4 hours, placed in a muffle furnace, calcined at 320 °C for 4 hours, and further calcined at 750 °C for 4 hours to obtain a catalyst product, of which the composition is shown in Table 1.
The catalyst was evaluated and analyzed as described in Example 1, the results of the alkali content analysis and the strength measurement are shown in Table 4, and the evaluation results are shown in Table 5.

Example 9

52.62 parts by weight of iron oxide red, calculated as $Fe_2O_3$, 13.45 parts by weight of iron oxide yellow, calculated as $Fe_2O_3$, 4.66 parts by weight of potassium carbonate, calculated as $K_2O$, 10.55 parts by weight of cerium hydroxide, calculated as $CeO_2$, 1.72 parts by weight of ammonium tungstate, calculated as $WO_3$, 4.83 parts by weight of strontium carbonate, calculated as $SrO$, 4.4 parts by weight of $GeO_2$, 0.12 parts by weight of $HfO_2$ and 5.69 parts by weight of sodium carboxymethyl cellulose were stirred in a kneader for 0.4 hours, 7.65 parts by weight of $ZnFe_2O_4$ was added thereto and further stirred for 1 hour, deionized water was added thereto in an amount of 35 wt% relative to the total weight of the catalyst raw materials, blended for 1 hour, the resultant was taken out and extruded into granules having a diameter of 3 mm and a length of 6 mm, the granules were placed in an oven, dried at 95 °C for 0.5 hours and further dried at 145 °C for 0.5 hours, then placed in a muffle furnace, calcined at 750 °C for 1 hour, and further calcined at 960 °C for 1 hour to obtain a catalyst product, of which the composition is shown in Table 1.
The catalyst was evaluated and analyzed as described in Example 1, the results of the alkali content analysis and the strength measurement are shown in Table 4, and the evaluation results are shown in Table 5.

Example 10

55.36 parts by weight of iron oxide red, calculated as $Fe_2O_3$, 17.42 parts by weight of iron oxide yellow, calculated as $Fe_2O_3$, 3.71 parts by weight of potassium carbonate, calculated as $K_2O$, 9.01 parts by weight of cerium acetate, calculated as $CeO_2$, 4.82 parts by weight of ammonium tungstate, calculated as $WO_3$, 1.83 parts by weight of strontium carbonate, calculated as $SrO$, 4.58 parts by weight of $GeO_2$, 0.45 parts by weight of $HfO_2$, and 5.69 parts by weight of sodium carboxymethyl cellulose were stirred in a kneader for 0.5 hours, 2.82 parts by weight of $ZnFe_2O_4$ were added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, then placed in a muffle furnace, calcined at 455 °C for 3 hours and further calcined at 930 °C for 3 hours to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.

Example 11

60.69 parts by weight of iron oxide red, calculated as $Fe_2O_3$, 17.36 parts by weight of iron oxide yellow, calculated as $Fe_2O_3$, 5.05 parts by weight of potassium carbonate, calculated as $K_2O$, 6.15 parts by weight of cerium acetate, calculated as $CeO_2$, 1.78 parts by weight of ammonium tungstate, calculated as $WO_3$, 0.55 parts by weight of strontium carbonate, calculated as SrO, 2.02 parts by weight of $GeO_2$, 0.25 parts by weight of $HfO_2$ and 5.69 parts by weight of sodium carboxymethyl cellulose were stirred in a kneader for 0.5 hours, 6.15 parts by weight of $ZnFeO_{24}$ was added thereto and further stirred for 1.5 hours, deionized water was added thereto in an amount of 26.3 wt% relative to the total weight of the catalyst raw materials, and blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, then placed in a muffle furnace, calcined at 455 °C for 3 hours, and further calcined at 930 °C for 3 hours, to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.

Comparative Example 5

54.4 parts by weight of iron oxide red, calculated as $Fe_2O_3$, 21.1 parts by weight of iron oxide yellow, calculated as $Fe_2O_3$, 4.77 parts by weight of potassium carbonate, calculated as $K_2O$, 4.55 parts by weight of $K_2SiO_3$, 7.75 parts by weight of cerium acetate, calculated as $CeO_2$, 2.58 parts by weight of ammonium tungstate, calculated as $WO_3$, 1.38 parts by weight of strontium carbonate, calculated as SrO, 1.48 parts by weight of $GeO_2$, 0.02 parts by weight of $HfO_2$ and 5.69 parts by weight of sodium carboxymethyl cellulose were stirred in a kneader for 0.5 hours, 3.74 parts by weight of $ZnFe_2O_4$ were added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, placed in a muffle furnace, calcined at 455 °C for 3 hours, and further calcined at 930 °C for 3 hours, to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.

Comparative Example 6

42.9 parts by weight of iron oxide red, calculated as $Fe_2O_3$, 25.8 parts by weight of iron oxide yellow, calculated as $Fe_2O_3$, 5.8 parts by weight of potassium carbonate, calculated as $K_2O$, 9.1 parts by weight of cerium acetate, calculated as $CeO_2$, 2.5 parts by weight of ammonium tungstate, calculated as $WO_3$, 4.1 parts by weight of strontium carbonate, calculated as SrO, 4.77 parts by weight of $GeO_2$, 0.13 parts by weight of $HfO_2$ and 5.69 parts by weight of sodium carboxymethyl cellulose were stirred in a kneader for 0.5 hours, 4.9 parts by weight of $ZnFeO_{24}$ was added thereto and stirred for 1.5 hours, deionized water was added thereto in an amount of 26.3 wt% relative to the total weight of the catalyst raw materials, and blended for 0.5 hours, the resultant was taken out and extruded into granules having a diameter of 3 mm and a length of 6 mm, and the granules were placed in an oven, dried at 55 °C for 2 hours, and further dried at 135 °C for 3 hours, then placed in a muffle furnace, calcined at 455 °C for 3 hours and further calcined at 930 °C for 3 hours, to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.

Comparative Example 7

54.8 parts by weight of iron oxide red, calculated as $Fe_2O_3$, 16.04 parts by weight of iron oxide yellow, calculated as $Fe_2O_3$, 5.85 parts by weight of potassium carbonate, calculated as $K_2O$, 7.11 parts by weight of $CeO_2$, 4.42 parts by weight of ammonium tungstate, calculated as $WO_3$, 3.35 parts by weight of strontium carbonate, calculated as SrO, 3.85 parts by weight of $GeO_2$, 0.99 parts by weight of $MoO_3$, 0.09 parts by weight of $HfO_2$ and 5.69 parts by weight of sodium carboxymethyl cellulose were stirred in a kneader for 0.5 hours, 3.5 parts by weight of $ZnFe_2O_4$ were added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, then placed in a muffle furnace, calcined at 455 °C for 3 hours, and further dried at 930 °C for 3 hours, to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.


Comparative Example 8

50.5 parts by weight of iron oxide red, calculated as $Fe_2O_3$, 17.01 parts by weight of iron oxide yellow, calculated as $Fe_2O_3$, 4.66 parts by weight of potassium carbonate, calculated as $K_2O$, 6.15 parts by weight of cerium acetate, calculated as $CeO_2$, 3.29 parts by weight of ammonium tungstate, calculated as $WO_3$, 2.73 parts by weight of strontium carbonate, calculated as SrO, 4.53 parts by weight of $GeO_2$, 0.75 parts by weight of $HfO_2$, 2.1 parts by weight of cement, and 5.69 parts by weight of sodium carboxymethyl cellulose were stirred in a kneader for 0.5 hours, 8.28 parts by weight of $ZnFeO_{24}$ was added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, then placed in a muffle furnace, calcined at 455 °C for 3 hours, and further calcined at 930 °C for 3 hours, to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.


Example 12

48.01 parts by weight of iron oxide red, calculated as $Fe_2O_3$, 20.4 parts by weight of iron oxide yellow, calculated as $Fe_2O_3$, 5.35 parts by weight of potassium carbonate, calculated as $K_2O$, 11.15 parts by weight of cerium acetate, calculated as $CeO_2$, 0.91 parts by weight of ammonium tungstate, calculated as $WO_3$, 4.85 parts by weight of strontium carbonate, calculated as SrO, 4.4 parts by weight of $SnO_2$, 0.28 parts by weight of $HfO_2$ and 5.69 parts by weight of sodium carboxymethyl cellulose were stirred for 0.5 hours, 4.65 parts by weight of $ZnFe_2O_4$ were added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried 55 °C for 2 hours, and further dried at 135 °C for 3 hours, then placed in a muffle furnace, calcined at 455 °C for 3 hours, and further calcined at 930 °C for 3 hours to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.


Example 13

52.7 parts by weight of iron oxide red, calculated as $Fe_2O_3$, 25.2 parts by weight of iron oxide yellow, calculated as $Fe_2O_3$, 5.05 parts by weight of potassium carbonate, calculated as $K_2O$, 8.99 parts by weight of cerium acetate, calculated as $CeO_2$, 2.58 parts by weight of ammonium tungstate, calculated as $WO_3$, 1.38 parts by weight of strontium carbonate, calculated as SrO, 2.89 parts by weight of $PbO_2$, 0.26 parts by weight of $HfO_2$ and 5.69 parts by weight of sodium carboxymethyl cellulose were stirred in a kneader for 0.5 hours, 0.95 parts by weight of $ZnFeO_{24}$ was added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, then placed in a muffle furnace, calcined at 455 °C for 3 hours, and further calcined at 930 °C for 3 hours to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.


Example 14

54.25 parts by weight of iron oxide red, calculated as $Fe_2O_3$, 18.42 parts by weight of iron oxide yellow, calculated as $Fe_2O_3$, 5.19 parts by weight of potassium carbonate, calculated as $K_2O$, 9.15 parts by weight of cerium acetate, calculated as $CeO_2$, 3.22 parts by weight of ammonium tungstate, calculated as $WO_3$, 2.69 parts by weight of strontium carbonate, calculated as SrO, 3.95 parts by weight of $GeO_2$, 0.48 parts by weight of $HfO_2$ and 5.69 parts by weight of sodium carboxymethyl cellulose were stirred in a kneader for 0.5 hours, 2.65 parts by weight of $NiFe_2O_4$ were added thereto and further stirred for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 55 °C for 2 hours, and further dried at 135 °C for 3 hours, then placed in a muffle furnace, calcined at 455 °C for 3 hours and further calcined at 930 °C for 3 hours to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.

Comparative Example 9

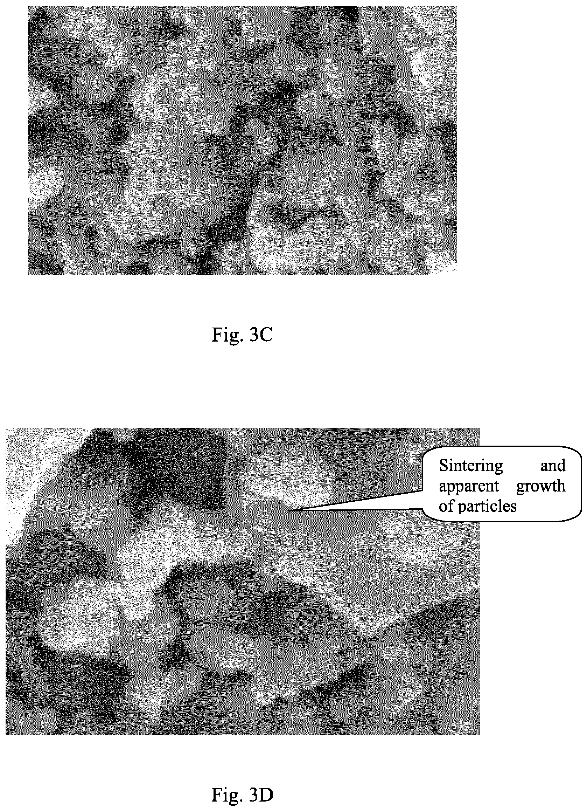

A catalyst was prepared as described in Example 1, except that $ZnFe_2O_4$ was stirred together with iron oxide red, iron oxide yellow, potassium carbonate, cerium acetate, ammonium tungstate, strontium carbonate, $GeO_2$ and $HfO_2$ and sodium carboxymethyl cellulose for 2 hours, deionized water was added thereto in an amount of 26.3 wt% relative to the total weight of the catalyst raw materials, stirred and mixed for 0.5 hours, and the proportions of other components added and other preparation steps were the same as in Example 1. The $H_2$-TPR pattern of the resulting catalyst is shown in Fig. 2, the electron microscope images of the catalyst before and after reaction are shown in Fig. 3C and Fig. 3D, respectively, and the analysis method is the same as in Example 1.

As can be seen from Fig. 2, the reduction completion temperature of the catalyst obtained in Comparative Example 9 was 674 °C, and the reduction completion temperature of the catalyst obtained in Example 1 was 72 °C higher than that of Comparative Example 9, indicating that the catalyst obtained in Example 1 has better reduction resistance.

As can be seen from Figs. 3A and 3B, after 1500 hours of reaction, the surface particles of the catalyst obtained in Example 1 still have clear contours and are uniformly distributed; in contrast, as can be seen from Figs. 3C and 3D, the catalyst obtained in Comparative Example 9 shows a phenomenon of sintering and obvious growth of the particles on the surface after reaction, which indicates that the catalyst obtained in Example 1 has better stability.

The catalyst was evaluated and analyzed as described in Example 1, the results of the alkali content analysis and the strength measurement are shown in Table 4, and the evaluation results are shown in Table 5.

Comparative Example 10

A catalyst was prepared as described in Example 1, except that 8.88 parts by weight of cerium acetate, calculated as $CeO_2$, was added, and at the same time magnesium hydroxide was also added in an amount equivalent to 1.01 parts by weight of MgO, and the proportions of other components added and other preparation steps were the same as in Example 1.

The catalyst was evaluated and analyzed as described in Example 1, the results of the alkali content analysis and the strength measurement are shown in Table 4, and the evaluation results are shown in Table 5.

Comparative Example 11

54.4 parts by weight of iron oxide red, calculated as $Fe_2O_3$, 21.1 parts by weight of iron oxide yellow, calculated as $Fe_2O_3$, 2.61 parts by weight of potassium carbonate, calculated as $K_2O$, 4.55 parts by weight of $K_2ZnO_2$, 10.26 parts by weight of cerium acetate, calculated as $CeO_2$, 2.58 parts by weight of ammonium tungstate, calculated as $WO_3$, 1.38 parts by weight of strontium carbonate, calculated as SrO, 3.74 parts by weight of $ZnFe_2O_4$, 1.48 parts by weight of $GeO_2$, 0.02 parts by weight of $HfO_2$ and 5.69 parts by weight of sodium carboxymethyl cellulose were stirred in a kneader for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 60 °C for 2 hours, and further dried at 130 °C for 3 hours, then placed in a muffle furnace, calcined at 650 °C for 3 hours, and further calcined at 920 °C for 3 hours, to obtain a catalyst product, of which the composition is shown in Table 2.

The catalyst was evaluated and analyzed as described in Example 1, the results of the alkali content analysis and the strength measurement are shown in Table 4, and the evaluation results are shown in Table 5.

Comparative Example 12

49.7 parts by weight of iron oxide red, calculated as $Fe_2O_3$, 19.5 parts by weight of iron oxide yellow, calculated as $Fe_2O_3$, 5.81 parts by weight of potassium carbonate, calculated as $K_2O$, 5.73 parts by weight of $K_2SiO_3$, 11.23 parts by weight of cerium nitrate, calculated as $CeO_2$, 0.74 parts by weight of ammonium tungstate, calculated as $WO_3$, 4.27

parts by weight of magnesium hydroxide, calculated as MgO, 0.85 parts by weight of SrO, 1.98 parts by weight of $Eu_2O_3$, 0.19 parts by weight of $GeO_2$, and 5.69 parts by weight of graphite were stirred in a kneader for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 60 °C for 2 hours and further dried at 130 °C for 3 hours, then placed in a muffle furnace, calcined at 650 °C for 3 hours, and further calcined at 920 °C for 3 hours, to obtain a catalyst product, of which the composition is shown in Table 3.

The catalyst was evaluated and analyzed as described in Example 1, the results of the alkali content analysis and the strength measurement are shown in Table 4, and the evaluation results are shown in Table 5.

Comparative Example 13

50.2 parts by weight of iron oxide red, calculated as $Fe_2O_3$, 19.0 parts by weight of iron oxide yellow, calculated as $Fe_2O_3$, 5.81 parts by weight of potassium carbonate, calculated as $K_2O$, 5.73 parts by weight of $K_2ZnO_2$, 11.23 parts by weight of cerium nitrate, calculated as $CeO_2$, 0.74 parts by weight of ammonium tungstate, calculated as $WO_3$, 4.27 parts by weight of magnesium hydroxide, calculated as MgO, 0.85 parts by weight of SrO, 1.98 parts by weight of $Eu_2O_3$, 0.19 parts by weight of $GeO_2$, and 5.69 parts by weight of graphite were stirred in a kneader for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 60 °C for 2 hours, and further dried at 130 °C for 3 hours, then placed in a muffle furnace, calcined at 650 °C for 3 hours, and further calcined at 920 °C for 3 hours, to obtain a catalyst product, of which the composition is shown in Table 3.

The catalyst was evaluated and analyzed as described in Example 1, the results of the alkali content analysis and the strength measurement are shown in Table 4, and the evaluation results are shown in Table 5.

Comparative Example 14

56.48 parts by weight of iron oxide red, calculated as $Fe_2O_3$, 19.17 parts by weight of iron oxide yellow, calculated as $Fe_2O_3$, 8.6 parts by weight of potassium carbonate, calculated as $K_2O$, 7.21 parts by weight of $CeO_2$, 2.58 parts by weight of ammonium tungstate, calculated as $WO_3$, 1.12 parts by weight of magnesium hydroxide, calculated as MgO, 4.84 parts by weight of $ZnFe_2O_4$ and 5.69 parts by weight of sodium carboxymethyl cellulose were stirred in a kneader for 1.5 hours, deionized water was added thereto in an amount of 26.3 wt% relative to the total weight of the catalyst raw materials, blended for 0.5 hours, the resultant was taken out and extruded into granules having a diameter of 3 mm and a length of 6 mm, the granules were placed in an oven, dried at 60 °C for 2 hours, and further dried at 130 °C for 3 hours, then placed in a muffle furnace, calcined at 650 °C for 3 hours, and further calcined at 920 °C for 3 hours, to obtain a catalyst product, of which the composition is shown in Table 3.

The catalyst was evaluated and analyzed as described in Example 1, the results of the alkali content analysis and the strength measurement are shown in Table 4, and the evaluation results are shown in Table 5.

Table 1 Composition of the catalysts obtained in the examples and comparative examples

| Composition, wt% | Ex. 1 | Comp. Ex. 1 | Comp. Ex. 2 | Ex. 2 | Comp. Ex. 3 | Ex. 3 | Comp. Ex. 4 | Ex. 4 | Ex. 5 | Ex. 6 | Ex. 7 | Ex. 8 | Ex. 9 |
|---|---|---|---|---|---|---|---|---|---|---|---|---|---|
| $Fe_2O_3$ | 75.3 | 79.13 | 77.4 | 75.3 | 77.4 | 75.3 | 77.4 | 75.3 | 75.3 | 75.3 | 75.3 | 70.91 | 66.07 |
| $K_2O$ | 4.66 | 4.9 | 4.66 | 4.66 | 4.66 | 4.66 | 4.66 | 4.66 | 4.66 | 4.66 | 4.66 | 2.37 | 4.66 |
| $CeO_2$ | 9.89 | 10.39 | 9.89 | 9.89 | 9.89 | 9.89 | 9.89 | 9.89 | 9.89 | 9.89 | 9.89 | 11.84 | 10.55 |
| $WO_3$ | 2.61 | 2.74 | 2.61 | 2.61 | 2.61 | 2.61 | 2.61 | 2.61 | 2.61 | 2.61 | 2.61 | 4.16 | 1.72 |
| $SrO$ | 2.38 | 2.5 | 2.38 | 2.38 | 2.38 | 2.38 | 2.38 | 2.38 | 2.38 | 2.38 | 2.38 | 3.35 | 4.83 |
| $ZnFe_2O_4$ | 3.16 | 0 | 0 | 3.16 | 0 | 3.16 | 0 | 3.16 | 3.16 | 3.16 | 3.16 | 5.5 | 7.65 |
| $ZnO$ | 0 | 0 | 1.06 | 0 | 1.06 | 0 | 1.06 | 0 | 0 | 0 | 0 | 0 | 0 |
| $GeO_2$ | 1.68 | 0 | 1.68 | 0 | 0 | 0 | 0 | 0.84 | 0.84 | 0 | 0.56 | 0.85 | 4.4 |
| $SnO_2$ | 0 | 0 | 0 | 1.68 | 1.68 | 0 | 0 | 0.84 | 0 | 0.84 | 0.56 | 0 | 0 |
| $PbO_2$ | 0 | 0 | 0 | 0 | 0 | 1.68 | 1.68 | 0 | 0.84 | 0.84 | 0.56 | 0 | 0 |
| $HfO_2$ | 0.32 | 0.34 | 0.32 | 0.32 | 0.32 | 0.32 | 0.32 | 0.32 | 0.32 | 0.32 | 0.32 | 0.06 | 0.12 |
| $Sb_2O_5$ | 0 | 0 | 0 | 0 | 0 | 0 | 0 | 0 | 0 | 0 | 0 | 0.96 | 0 |

Table 2 Composition of the catalysts obtained in the examples and comparative examples

| Composition, wt% | Ex. 10 | Ex. 11 | Comp. Ex. 5 | Comp. Ex. 6 | Comp. Ex. 7 | Comp. Ex. 8 | Ex. 12 | Ex. 13 | Ex. 14 | Comp. Ex. 9 | Comp. Ex. 10 | Comp. Ex. 11 |
|---|---|---|---|---|---|---|---|---|---|---|---|---|
| $Fe_2O_3$ | 72.78 | 78.05 | 75.5 | 68.7 | 70.84 | 67.51 | 68.41 | 77.9 | 72.67 | 75.3 | 75.3 | 75.5 |
| $K_2O$ | 3.71 | 5.05 | 4.77 | 5.8 | 5.85 | 4.66 | 5.35 | 5.05 | 5.19 | 4.66 | 4.66 | 2.61 |
| $K_2SiO_3$ | 0 | 0 | 4.55 | 0 | 0 | 0 | 0 | 0 | 0 | 0 | 0 | 0 |
| $K_2ZnO_2$ | 0 | 0 | 0 | 0 | 0 | 0 | 0 | 0 | 0 | 0 | 0 | 4.55 |
| $CeO_2$ | 9.01 | 6.15 | 7.75 | 9.1 | 7.11 | 6.15 | 11.15 | 8.99 | 9.15 | 9.89 | 8.88 | 10.26 |
| $WO_3$ | 4.82 | 1.78 | 2.58 | 2.5 | 4.42 | 3.29 | 0.91 | 2.58 | 3.22 | 2.61 | 2.61 | 2.58 |
| $SrO$ | 1.83 | 0.55 | 1.38 | 4.1 | 3.35 | 2.73 | 4.85 | 1.38 | 2.69 | 2.38 | 2.38 | 1.38 |
| $ZnFe_2O_4$ | 2.82 | 6.15 | 3.74 | 4.9 | 3.5 | 8.28 | 4.65 | 0.95 | 0 | 3.16 | 3.16 | 3.74 |
| $NiFe_2O_4$ | 0 | 0 | 0 | 0 | 0 | 0 | 0 | 0 | 2.65 | 0 | 0 | 0 |
| $GeO_2$ | 4.58 | 2.02 | 1.48 | 4.77 | 3.85 | 4.53 | 0 | 0 | 3.95 | 1.68 | 0 | 1.48 |
| $SnO_2$ | 0 | 0 | 0 | 0 | 0 | 0 | 4.4 | 0 | 0 | 0 | 1.68 | 0 |
| $PbO_2$ | 0 | 0 | 0 | 0 | 0 | 0 | 0 | 2.89 | 2.89 | 0 | 0 | 0 |
| $HfO_2$ | 0.45 | 0.25 | 0.02 | 0.13 | 0.09 | 0.75 | 0.28 | 0.26 | 0.48 | 0.32 | 0 | 0.02 |
| $Sb_2O_5$ | 0 | 0 | 0 | 0 | 0 | 0 | 0 | 0 | 0 | 0 | 0.32 | 0 |
| $MoO_3$ | 0 | 0 | 0 | 0 | 0.99 | 0 | 0 | 0 | 0 | 0 | 0 | 0 |
| Cement | 0 | 0 | 0 | 0 | 0 | 2.1 | 0 | 0 | 0 | 0 | 0 | 0 |
| $MgO$ | 0 | 0 | 0 | 0 | 0 | 0 | 0 | 0 | 0 | 0 | 1.01 | 0 |

Table 3 Composition of the catalysts obtained in the examples and comparative examples

| Composition, wt% | Comp. Ex. 12 | Comp. Ex. 13 | Comp. Ex. 14 |
|---|---|---|---|
| $Fe_2O_3$ | 69.2 | 69.2 | 75.65 |
| $K_2O$ | 5.81 | 5.81 | 8.6 |
| $K_2SiO_3$ | 5.73 | 0 | 0 |
| $K_2ZnO_3$ | 0 | 5.73 | 0 |
| $CeO_2$ | 11.23 | 11.23 | 7.21 |
| $WO_3$ | 0.74 | 0.74 | 2.58 |
| MgO | 4.27 | 4.27 | 1.12 |
| SrO | 0.85 | 0.85 | 0 |
| $Eu_2O_3$ | 1.98 | 1.98 | 0 |
| $GeO_2$ | 0.19 | 0.19 | 0 |
| $ZnFe_2O_4$ | 0 | 0 | 4.84 |

Table 4 Change in the properties of the catalysts obtained in the examples and comparative examples before and after reaction

| Catalyst | Before reaction | | | After reaction | | | | | |
|---|---|---|---|---|---|---|---|---|---|
| | Strong alkali content, mmol/g | Total alkali content, mmol/g | Strength, Kg/mm | Strong alkali content, mmol/g | Total alkali content, mmol/g | Strength, Kg/mm | Retention rate of strong alkali content, % | Retention rate of total alkali content, % | Retention rate of the strength, % |
| Ex. 1 | 0.066 | 0.371 | 3.35 | 0.057 | 0.334 | 2.88 | 86.36 | 90.03 | 85.97 |
| Comp. Ex. 1 | 0.044 | 0.237 | 1.55 | 0.022 | 0.121 | 0.76 | 50.0 | 51.05 | 49.03 |
| Comp. Ex. 2 | 0.052 | 0.286 | 1.76 | 0.029 | 0.160 | 0.96 | 55.77 | 55.94 | 54.55 |
| Ex. 2 | 0.068 | 0.371 | 3.31 | 0.059 | 0.334 | 2.89 | 86.76 | 90.03 | 87.31 |
| Comp. Ex. 3 | 0.055 | 0.306 | 1.82 | 0.031 | 0.177 | 1.04 | 56.36 | 57.84 | 57.14 |
| Ex. 3 | 0.065 | 0.376 | 3.39 | 0.056 | 0.338 | 2.92 | 86.15 | 89.89 | 86.14 |
| Comp. Ex. 4 | 0.056 | 0.313 | 1.89 | 0.032 | 0.185 | 1.1 | 57.14 | 59.11 | 58.2 |
| Ex. 4 | 0.076 | 0.384 | 3.52 | 0.067 | 0.353 | 3.15 | 88.16 | 91.93 | 89.49 |
| Ex. 5 | 0.072 | 0.382 | 3.61 | 0.064 | 0.348 | 3.22 | 88.89 | 91.1 | 89.20 |
| Ex. 6 | 0.074 | 0.385 | 3.56 | 0.066 | 0.352 | 3.15 | 89.19 | 91.43 | 88.48 |
| Ex. 7 | 0.079 | 0.397 | 3.88 | 0.073 | 0.377 | 3.68 | 92.41 | 94.96 | 94.85 |
| Ex. 8 | 0.067 | 0.366 | 3.38 | 0.058 | 0.330 | 2.94 | 86.57 | 90.16 | 86.98 |
| Ex. 9 | 0.061 | 0.324 | 3.21 | 0.052 | 0.285 | 2.71 | 85.25 | 87.96 | 84.42 |
| Ex. 10 | 0.064 | 0.339 | 3.18 | 0.055 | 0.306 | 2.76 | 85.94 | 90.27 | 86.79 |
| Ex. 11 | 0.063 | 0.335 | 3.26 | 0.054 | 0.296 | 2.76 | 85.71 | 88.36 | 84.66 |
| Comp. Ex. 5 | 0.099 | 0.593 | 2.12 | 0.059 | 0.362 | 1.21 | 59.60 | 61.05 | 57.08 |
| Comp. Ex. 6 | 0.058 | 0.317 | 2.32 | 0.036 | 0.200 | 1.42 | 62.07 | 63.09 | 61.21 |
| Comp. Ex. 7 | 0.052 | 0.305 | 2.45 | 0.034 | 0.204 | 1.56 | 65.38 | 66.89 | 63.67 |

| Catalyst | Before reaction | | | After reaction | | | | | |
|---|---|---|---|---|---|---|---|---|---|
| | Strong alkali content, mmol/g | Total alkali content, mmol/g | Strength, Kg/mm | Strong alkali content, mmol/g | Total alkali content, mmol/g | Strength, Kg/mm | Retention rate of strong alkali content, % | Retention rate of total alkali content, % | Retention rate of the strength, % |
| Comp. Ex. 8 | 0.046 | 0.267 | 2.98 | 0.027 | 0.160 | 1.76 | 58.70 | 59.93 | 59.06 |
| Ex. 12 | 0.068 | 0.36 | 3.41 | 0.059 | 0.324 | 2.98 | 86.76 | 90.0 | 87.39 |
| Ex. 13 | 0.067 | 0.379 | 3.11 | 0.056 | 0.323 | 2.62 | 83.58 | 85.22 | 84.24 |
| Ex. 14 | 0.062 | 0.337 | 3.12 | 0.053 | 0.303 | 2.66 | 85.48 | 89.91 | 85.27 |
| Comp. Ex. 9 | 0.06 | 0.319 | 2.76 | 0.041 | 0.223 | 1.87 | 68.33 | 69.91 | 67.75 |
| Comp. Ex. 10 | 0.094 | 0.587 | 2.45 | 0.058 | 0.370 | 1.46 | 61.70 | 63.03 | 59.59 |
| Comp. Ex. 11 | 0.056 | 0.317 | 1.77 | 0.034 | 0.194 | 1.05 | 60.71 | 61.20 | 59.32 |
| Comp. Ex. 12 | 0.083 | 0.473 | 2.32 | 0.052 | 0.305 | 1.47 | 62.65 | 64.48 | 63.36 |
| Comp. Ex. 13 | 0.091 | 0.517 | 2.16 | 0.055 | 0.321 | 1.33 | 60.44 | 62.09 | 61.57 |
| Comp. Ex. 14 | 0.052 | 0.301 | 2.28 | 0.036 | 0.233 | 1.59 | 69.23 | 77.41 | 69.74 |

Table 5 Measured results of the catalytic performance of the catalysts obtained in the examples and comparative examples

| Catalyst | Reaction time | | | | | |
| | 100 hours | | | 1500 hours | | |
| | Content of benzene and toluene in the product, wt% | Conversion-of ethylbenzene, % | Selectivity of styrene, % | Content of benzene and toluene in the product, wt% | Conversion of ethylbenzene, % | Selectivity of styrene, % |
|---|---|---|---|---|---|---|
| Ex. 1 | 2.74 | 76.65 | 96.35 | 2.54 | 74.15 | 96.85 |
| Comp. Ex. 1 | 5.11 | 69.94 | 91.35 | 5.07 | 57.44 | 91.45 |
| Comp. Ex. 2 | 4.81 | 71.17 | 92.07 | 4.71 | 60.67 | 92.32 |
| Ex. 2 | 2.86 | 76.5 | 96.21 | 2.65 | 73.9 | 96.73 |
| Comp. Ex. 3 | 4.66 | 71.1 | 92.31 | 4.55 | 61.3 | 92.59 |
| Ex. 3 | 2.82 | 76.56 | 96.24 | 2.64 | 73.86 | 96.69 |
| Comp. Ex. 4 | 4.75 | 71.21 | 92.15 | 4.65 | 61.36 | 92.39 |
| Ex. 4 | 2.5 | 77.1 | 96.75 | 2.24 | 75.2 | 97.4 |
| Ex. 5 | 2.57 | 77.2 | 96.67 | 2.29 | 75.25 | 97.37 |
| Ex. 6 | 2.55 | 77.23 | 96.71 | 2.29 | 75.29 | 97.36 |
| Ex. 7 | 2.26 | 77.6 | 97.17 | 1.93 | 76.7 | 97.99 |
| Ex. 8 | 2.68 | 76.51 | 96.45 | 2.47 | 73.86 | 96.97 |
| Ex. 9 | 3.45 | 75.18 | 94.76 | 3.27 | 68.68 | 95.15 |
| Ex. 10 | 3.51 | 76.17 | 95.22 | 3.32 | 73.49 | 95.7 |
| Ex. 11 | 3.2 | 76.24 | 95.65 | 3.01 | 73.66 | 96.13 |
| Comp. Ex. 5 | 4.41 | 72.6 | 92.93 | 4.29 | 65.1 | 93.22 |
| Comp. Ex. 6 | 4.17 | 73.2 | 93.54 | 4.05 | 64.4 | 93.85 |
| Comp. Ex. 7 | 4.25 | 71.0 | 93.14 | 4.14 | 59.8 | 93.41 |
| Comp. Ex. 8 | 4.61 | 71.37 | 92.52 | 4.54 | 60.47 | 92.7 |
| Ex. 12 | 2.91 | 76.56 | 96.06 | 2.72 | 73.66 | 96.54 |
| Ex. 13 | 3.59 | 75.67 | 95.04 | 3.41 | 72.37 | 95.5 |
| Ex. 14 | 3.55 | 76.09 | 95.13 | 3.37 | 73.32 | 95.56 |
| Comp. Ex. 9 | 3.85 | 71.8 | 93.92 | 3.69 | 65.30 | 94.31 |
| Comp. Ex. 10 | 4.63 | 70.58 | 91.32 | 4.5 | 65.08 | 91.64 |
| Comp. Ex. 11 | 4.43 | 72.58 | 92.9 | 4.3 | 65.28 | 93.22 |

(continued)

| Catalyst | Reaction time | | | | | |
| --- | --- | --- | --- | --- | --- | --- |
| | 100 hours | | | 1500 hours | | |
| | Content of benzene and toluene in the product, wt% | Conversion-of ethylbenzene, % | Selectivity of styrene, % | Content of benzene and toluene in the product, wt% | Conversion of ethylbenzene, % | Selectivity of styrene, % |
| Comp. Ex. 12 | 3.57 | 71.93 | 93.56 | 3.43 | 68.02 | 93.89 |
| Comp. Ex. 13 | 3.71 | 71.07 | 93.39 | 3.59 | 66.23 | 93.65 |
| Comp. Ex. 14 | 4.16 | 72.11 | 92.54 | 4.01 | 67.89 | 92.93 |

As can be seen the measured results of the above examples and comparative examples, compared with the catalysts of the comparative examples and the prior arts, the catalyst of the present application, by employing a specific coordination of elements and controlling the total alkali content in a range of 0.32-0.46 mmol/g and the strong alkali content in a range of 0.061-0.082 mmol/g, has outstanding advantages of high ethylbenzene conversion, less benzene and toluene by-products, high styrene selectivity, high stability in catalytic performance and strength, and high stability in alkali content before and after reaction, which is beneficial to reducing the cost and improving the efficiency of the styrene plant. Thus, the catalyst of the present application is a dehydrogenation catalyst satisfying the market requirements, and can be well used in industrial production of styrene by ethylbenzene dehydrogenation at a low reaction temperature and an ultralow steam-to-oil ratio.

The present application is illustrated in detail hereinabove with reference to preferred embodiments, but is not intended to be limited to those embodiments. Various modifications may be made following the inventive concept of the present application, and these modifications shall be within the scope of the present application.

It should be noted that the various technical features described in the above embodiments may be combined in any suitable manner without contradiction, and in order to avoid unnecessary repetition, various possible combinations are not described in the present application, but such combinations shall also be within the scope of the present application.

In addition, the various embodiments of the present application can be arbitrarily combined as long as the combination does not depart from the spirit of the present application, and such combined embodiments should be considered as the disclosure of the present application.

## Claims

1. An Iron-potassium-cerium-based composite oxide catalyst, comprising, in addition to metal elements Fe, K and Ce, a metal element M that is at least one selected from the group consisting of Group IIA metal elements, Group VIB metal elements other than Cr, and Group IVA metal elements, wherein the catalyst has a total alkali content of 0.32-0.46 mmol/g, preferably 0.32-0.42 mmol/g, more preferably 0.324-0.397 mmol/g, particularly preferably 0.384-0.397 mmol/g, and a strong alkali content 0.061-0.082 mmol/g, preferably 0.061-0.080 mmol/g, more preferably 0.061-0.079 mmol/g, particularly preferably 0.072-0.079 mmol/g.

2. The catalyst according to claim 1, wherein the metal element M is a combination of at least two selected from the group consisting of Group IIA metal elements, Group VIB metal elements other than Cr and Group IVA metal elements, preferably a combination of at least one Group IIA metal element, at least one Group VIB metal element other than Cr and at least one Group IVA metal element.

3. The catalyst according to claim 1 or 2, wherein the catalyst has at least one of the following characteristics:

the Group IIA metal element comprised in the catalyst is not Mg, but is preferably Sr;
the Group VIB metal element comprised in the catalyst is not Cr or Mo, but is preferably W;
the Group IVA metal element comprised in the catalyst is selected from the group consisting of Ge, Sn and Pb, or combinations thereof, and

the catalyst does not comprise a binder, such as montmorillonite, diatomite, cement, metahalloysite, saponite, kaolin, halloysite, hydrotalcite, sepiolite, rectorite, attapulgite, bentonite, or combinations thereof.

4. The catalyst according to any one of claims 1-3, wherein the catalyst has at least one of the following characteristics:

after 1500 hours of reaction under conditions including a pressure of -45 kPa, a mass space velocity of ethylbenzene of 0.75 h$^{-1}$, a temperature of 600 °C, and a weight ratio of water to ethylbenzene of 0.9, the retention rate of the crushing strength of the catalyst is 80% or higher;
after 1500 hours of reaction under conditions including a pressure of -45 kPa, a mass space velocity of ethylbenzene of 0.75 h$^{-1}$, a temperature of 600 °C, and a weight ratio of water to ethylbenzene of 0.9, the retention rate of the total alkali content of the catalyst is 82% or higher, and the retention rate of the strong alkali content of the catalyst is 80% or higher;
the catalyst has a reduction completion temperature of 730 °C or higher according to the H$_2$-TPR test.

5. The catalyst according to any one of claims 1-4, wherein the catalyst has a K$_2$O content of 2.3-6 wt%, preferably 2.3-5.5 wt%, based on the total amount of the catalyst.

6. The catalyst according to claim 5, wherein the catalyst has a Fe$_2$O$_3$ content of 66-80 wt%, preferably 67.5-79 wt%; a K$_2$O content of 2.3-6 wt%, preferably 2.3-5.5 wt%; a CeO$_2$ content of 6-12 wt%; and a content of the oxide of the metal element M of 2-16 wt%, based on the total amount of the catalyst;
preferably, the catalyst has a WO$_3$ content of 0.5-5 wt%, a SrO content of 0.5-5 wt%, and a content of the Group IVA metal oxide of 0.5-5 wt%, based on the total amount of the catalyst.

7. The catalyst according to any one of claims 1-6, wherein the catalyst further comprises 0.5-8 wt%, preferably 1-7 wt%, more preferably 2-6 wt%, of a ferrite, and the ferrite is preferably ZnFe$_2$O$_4$;
preferably, the catalyst further comprises 0.05-0.5 wt% of a Group IVB metal oxide, preferably HfO$_2$, and/or 0.5-1.5 wt% of a Group VA metal oxide, preferably Sb$_2$O$_5$, based on the total amount of the catalyst.

8. A method for producing the iron-potassium-cerium-based composite oxide catalyst according to any one of claims 1-7, comprising the steps of mixing an Fe source, a K source, a Ce source, an M source, optionally a source of a Group IVB metal element, optionally a source of a Group VA metal element, and optionally a ferrite, with a pore-forming agent and a solvent and shaping, optionally drying and/or calcining, to obtain the catalyst,
wherein the M source is at least one selected from the group consisting of sources of Group IIA metal elements, sources of Group VIB metal elements other than Cr, and sources of Group IVA metal elements, preferably at least one of a W source, an Sr source and a source of a Group IVA metal element, more preferably a combination of at least two of a W source, an Sr source and a source of a Group IVA metal element, particularly preferably a combination of a W source, an Sr source and at least one Group IVA metal element source.

9. The method according to claim 8, comprising the steps of:

1) mixing the Fe source, the K source, the Ce source, the M source, the optional Group IVB metal element source, and the optional Group VA metal element source with the pore-forming agent;
2) mixing the mixture obtained in step 1) with the ferrite; and
3) mixing the mixture obtained in step 2) with the solvent and shaping, optionally drying and/or calcining, to obtain the catalyst.

10. The method according to claim 8 or 9, wherein the method has at least one of the following features:

the Fe source is selected from iron oxide red, iron oxide yellow or a combination thereof, preferably a combination of the iron oxide red and the iron oxide yellow, and further preferably, the weight ratio of the iron oxide red to the iron oxide yellow, calculated as Fe$_2$O$_3$, is 2-4 : 1;
the Ce source is selected from cerium acetate, cerium hydroxide, or a combination thereof;
the K source is selected from potassium carbonate, potassium bicarbonate or a combination thereof;
the Group IIA metal element source is selected from salts of Group IIA metal elements, oxides of Group IIA metal elements, or combinations thereof, and preferably dose not comprise Mg;
the source of Group VIB metal element other than Cr is selected from salts of Group VIB metal elements, oxides of Group VIB metal elements, or combinations thereof, and preferably does not comprise Mo;
the W source is selected from ammonium tungstate, ammonium metatungstate, tungsten trioxide, or a combination thereof;

the Sr source is selected from strontium carbonate, strontium hydroxide or a combination thereof;

the source of Group IVA metal element is selected from salts of Group IVA metal elements, oxides of Group IVA metal elements, or a combination thereof, preferably the Group IVA metal element is selected from Ge, Sn and Pb, or a combination thereof;

the source of Group IVB metal element is selected from salts of Group IVB metal elements, oxides of Group IVB metal elements, or a combination thereof, preferably selected from Hf-containing salts, $HfO_2$, or a combination thereof;

the source of Group VA metal element is selected from salts of Group VA metal elements, oxides of Group VA metal elements or a combination thereof, preferably selected from Sb-containing salts, $Sb_2O_5$ or a combination thereof; and

the ferrite is $ZnFe_2O_4$.

11. The method according to any one of claims 8-10, wherein the method has at least one of the following characteristics:

the amount of the pore-forming agent added is 2.2-6.3 wt%, preferably 3.8-5.6 wt%, relative to the total amount of the Fe source, the K source, the Ce source, the M source, the optional ferrite, the optional Group IVB metal element source and the optional Group VA metal element source;

the pore-forming agent is selected from polystyrene, graphite, cellulose and derivatives thereof, or a combination thereof;

the amount of the solvent added is 15-35 wt%, preferably 22-32 wt%, relative to the total amount of the catalyst raw materials; and

the solvent is water.

12. The method according to any one of claims 8-11, wherein the method has at least one of the following characteristics;

the mixing time of step 1), step 2) and step 3) is independently 0.1-2h, preferably, the mixing time of step 1) is 0.1-0.6 h, the mixing time of step 2) is 1-2 h, and the mixing time of step 3) is 0.2-1 h;

the drying conditions of step 3) include: a temperature of 30-145 °C and a drying time of 1-8 h, and preferably, the drying comprises drying at 35-95 °C for 0.5-4 h, then heating to 95-145 °C, and further drying for 0.5-4 h; and

the calcining conditions of step 3) include: a temperature of 320-960 °C, and a calcining time of 2-8 h, preferably, the calcining comprises calcining at 320-750 °C for 1-4 h, then heating to 750-960 °C, and further calcining for 1-4 h.

13. Use of an iron-potassium-cerium-based composite oxide catalyst according to any one of claims 1 to 7 for the dehydrogenation of alkyl aromatic hydrocarbons, preferably the alkyl aromatic hydrocarbon is one or more of $C_8$-$C_{10}$ alkyl benzenes, more preferably ethylbenzene.

14. A process for the dehydrogenation of alkyl aromatics, comprising the step of contacting an alkyl aromatic hydrocarbon with the iron-potassium-cerium-based composite oxide catalyst according to any one of claims 1 to 7 for reaction under dehydrogenation conditions;

preferably, the dehydrogenation conditions include: a temperature of 580-620 °C, preferably 590-610 °C, a mass space velocity of the alkyl aromatic hydrocarbon of 0.5-1 $h^{-1}$, preferably 0.6-0.8 $h^{-1}$, a weight ratio of water to the alkyl aromatic hydrocarbon of 0.7-1, preferably 0.8-1, and a pressure of -60 kPa to atmospheric pressure, preferably -50 kPa to atmospheric pressure;

preferably, the alkyl aromatic hydrocarbon is one or more of $C_8$-$C_{10}$ alkyl benzenes, more preferably ethylbenzene.

Fig. 1

Fig. 2

Fig. 3A

Fig. 3B

Fig. 3C

Fig. 3D

## INTERNATIONAL SEARCH REPORT

| International application No. |
| --- |
| **PCT/CN2021/123478** |

**A. CLASSIFICATION OF SUBJECT MATTER**

B01J 23/888(2006.01)i; C07C 5/333(2006.01)i; C07C 15/46(2006.01)i; C07C 5/32(2006.01)i

According to International Patent Classification (IPC) or to both national classification and IPC

**B. FIELDS SEARCHED**

Minimum documentation searched (classification system followed by classification symbols)

B01J23/-; C07C5/-; C07C15/-

Documentation searched other than minimum documentation to the extent that such documents are included in the fields searched

Electronic data base consulted during the international search (name of data base and, where practicable, search terms used)

CNTXT, CNKI, 读秀, ENTXT, VEN, Elsevier: 铁, 氧化铁红, 氧化铁黄, Fe, Fe2O3, 钾, 氧化钾, K, K2O, 铈, 氧化铈, Ce, CeO2, 钼, 氧化钼, Mo, MoO2, 钨, 氧化钨, W, WO3, 铍, Be, 镁, 氧化镁, Mg, MgO, 钙, 氧化钙, Ca, CaO, 锶, 氧化锶, Sr, SrO, 钡, 氧化钡, Ba, BaO, 硅, 二氧化硅, Si, SiO2, 锡, 氧化锡, Sn, SnO2, 锗, 氧化锗, Ge, GeO2, 铅, 氧化铅, Pb, PbO2, 铁酸盐, 铁酸锌, ZnFe2O4, 铁酸锰, 铁酸铜, 铁酸镍, 铪, 氧化铪, Hf, HfO2, 锑, 氧化锑, Sb, Sb2O5, 钛, 氧化钛, Ti, TiO2, 致孔剂, 造孔剂, 碱量, 碱中心, 乙苯, 脱氢, 苯乙烯, ron, iron oxide red, yellow iron oxide, , potassium, potassium oxide, cerium, cerium oxide, molybdenum, molybdenum oxide, tungsten, tungsten oxide, beryllium, Be, magnesium, magnesium oxide, calcium, calcium oxide, strontium, strontium oxide, barium, barium oxide, silicon, silica, tin, tin oxide, germanium, germanium oxide, lead, lead oxide, Pb, PbO2, ferric acid salt, zinc ferrate, manganese ferrate, copper ferrate, nickel ferrate, hafnium, hafnium oxide, antimony, antimony oxide, Antimony, antimony oxide, titanium, titanium oxide, porogen, pore former, alkali amount, alkali center, ethylbenz+, dehydrogen+, styrene

**C. DOCUMENTS CONSIDERED TO BE RELEVANT**

| Category* | Citation of document, with indication, where appropriate, of the relevant passages | Relevant to claim No. |
| --- | --- | --- |
| X | CN 109569639 A (SINOPEC CORP.; SINOPEC SHANGHAI RESEARCH INSTITUTE OF PETROCHEMICAL TECHNOLOGY) 05 April 2019 (2019-04-05) description, paragraphs 7-37 | 1-14 |
| X | CN 101623642 A (SINOPEC CORP.; SINOPEC SHANGHAI RESEARCH INSTITUTE OF PETROCHEMICAL TECHNOLOGY) 13 January 2010 (2010-01-13) description, pages 2-3 | 1-14 |
| X | CN 103028419 A (SINOPEC CORP.; SINOPEC SHANGHAI RESEARCH INSTITUTE OF PETROCHEMICAL TECHNOLOGY) 10 April 2013 (2013-04-10) description, paragraphs 7-26 | 1-14 |

☑ Further documents are listed in the continuation of Box C.  ☑ See patent family annex.

```
*    Special categories of cited documents:
"A"  document defining the general state of the art which is not considered
     to be of particular relevance
"E"  earlier application or patent but published on or after the international
     filing date
"L"  document which may throw doubts on priority claim(s) or which is
     cited to establish the publication date of another citation or other
     special reason (as specified)
"O"  document referring to an oral disclosure, use, exhibition or other
     means
"P"  document published prior to the international filing date but later than
     the priority date claimed
```

```
"T"  later document published after the international filing date or priority
     date and not in conflict with the application but cited to understand the
     principle or theory underlying the invention
"X"  document of particular relevance; the claimed invention cannot be
     considered novel or cannot be considered to involve an inventive step
     when the document is taken alone
"Y"  document of particular relevance; the claimed invention cannot be
     considered to involve an inventive step when the document is
     combined with one or more such documents, such combination
     being obvious to a person skilled in the art
"&"  document member of the same patent family
```

| Date of the actual completion of the international search | Date of mailing of the international search report |
| --- | --- |
| **02 December 2021** | **10 January 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/123478**

### C. DOCUMENTS CONSIDERED TO BE RELEVANT

| Category* | Citation of document, with indication, where appropriate, of the relevant passages | Relevant to claim No. |
|---|---|---|
| X | CN 110681394 A (SINOPEC CORP.; SINOPEC SHANGHAI RESEARCH INSTITUTE OF PETROCHEMICAL TECHNOLOGY) 14 January 2020 (2020-01-14) description, paragraphs 7-36 | 1-14 |
| X | CN 102039204 A (SINOPEC CORP.; SINOPEC SHANGHAI RESEARCH INSTITUTE OF PETROCHEMICAL TECHNOLOGY) 04 May 2011 (2011-05-04) description, paragraphs 4-14 | 1-14 |
| X | CN 106582688 A (SINOPEC CORP.; SINOPEC SHANGHAI RESEARCH INSTITUTE OF PETROCHEMICAL TECHNOLOGY) 26 April 2017 (2017-04-26) description, paragraphs [0006]-[0033] | 1-14 |
| X | CN 106582685 A (SINOPEC CORP.; SINOPEC SHANGHAI RESEARCH INSTITUTE OF PETROCHEMICAL TECHNOLOGY) 26 April 2017 (2017-04-26) description, paragraphs 7-37 | 1-14 |
| A | CN 105233818 A (CHINA NATIONAL OFFSHORE OIL CORPORATION; CNOOC TIANJIN CHEMICAL RESEARCH & DESIGN INSTITUTE; CNOOC ENERGY TECHNOLOGY & SERVICES LIMITED) 13 January 2016 (2016-01-13) description, paragraphs 3-21 | 1-14 |

Form PCT/ISA/210 (second sheet) (January 2015)

INTERNATIONAL SEARCH REPORT
Information on patent family members

International application No.

**PCT/CN2021/123478**

| Patent document cited in search report | | | Publication date (day/month/year) | Patent family member(s) | | | Publication date (day/month/year) |
|---|---|---|---|---|---|---|---|
| CN | 109569639 | A | 05 April 2019 | CN | 109569639 | B | 09 June 2020 |
| CN | 101623642 | A | 13 January 2010 | CN | 101623642 | B | 27 April 2011 |
| CN | 103028419 | A | 10 April 2013 | CN | 103028419 | B | 26 November 2014 |
| CN | 110681394 | A | 14 January 2020 | | None | | |
| CN | 102039204 | A | 04 May 2011 | CN | 102039204 | B | 14 November 2012 |
| CN | 106582688 | A | 26 April 2017 | CN | 106582688 | B | 19 February 2019 |
| CN | 106582685 | A | 26 April 2017 | CN | 106582685 | B | 12 April 2019 |
| CN | 105233818 | A | 13 January 2016 | | None | | |

Form PCT/ISA/210 (patent family annex) (January 2015)

**EP 4 219 004 A1**

**REFERENCES CITED IN THE DESCRIPTION**

**Patent documents cited in the description**

- CN 202011099425
- CN 104096568 A
- US 5190906 A
- US 4804799 A
- US 6177602 A